(19) Europäisches Patentamt
European Patent Office
Office européen des brevets

(11) Numéro de publication: **0 033 255**
**B1**

(12) # FASCICULE DE BREVET EUROPEEN

(45) Date de publication du fascicule du brevet: **29.08.84**

(51) Int. Cl.³: **C 07 H 17/08,** A 61 K 31/70

(21) Numéro de dépôt: **81400026.1**

(22) Date de dépôt: **09.01.81**

(54) **Nouvelles oximes dérivées de l'érythromycine A, leur préparation, leur application comme médicaments et les compositions pharmaceutiques les renfermant.**

(30) Priorité: **11.01.80 FR 8000566**

(43) Date de publication de la demande:
**05.08.81 Bulletin 81/31**

(45) Mention de la délivrance du brevet:
**29.08.84 Bulletin 84/35**

(84) Etats contractants désignés:
**AT BE CH DE FR GB IT LI LU NL SE**

(56) Documents cités:
**EP - A - 0 001 981**
**DE - A - 2 515 076**
**DE - A - 2 515 077**
**FR - A - 2 201 874**

**CHEMICAL ABSTRACTS, vol. 87, no. 13, 26 septembre 1977, réf. 102607s, page 657 Columbus, Ohio, US**
**CHEMICAL ABSTRACTS, vol. 91, no. 15, 8 octobre 1979, réf. 123967g, page 632 Columbus, Ohio, US**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Gouin d'Ambrieres, Solange, 119, rue Manin, F-75019-Paris (FR)**
Inventeur: **Lutz, André, 20, rue du Général Uhrich, F-67000-Strasbourg (FR)**
Inventeur: **Gasc, Jean-Claude, 6, Place Georges Lyssandre, F-93140-Bondy (FR)**

(74) Mandataire: **Bourgouin, André et al, ROUSSEL-UCLAF Boîte postale no 9, 102, route de Noisy F-93230 Romainville (FR)**

**EP 0 033 255 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouvelles oximes dérivées de l'érythromycine A, leur préparation, leur application comme médicaments et les compositions pharmaceutiques les renfermant.

L'invention a pour objet les produits dérivés de l'érythromycine de formule

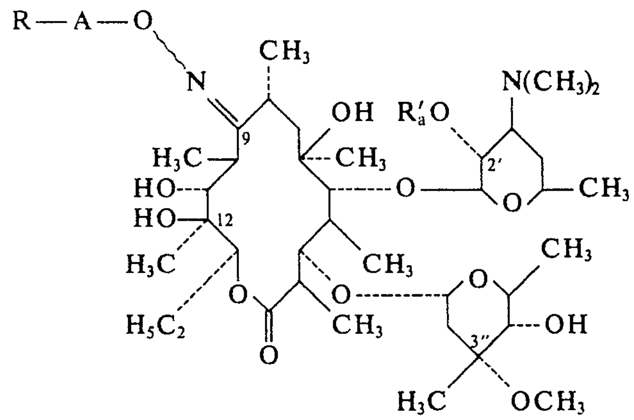

(I)

dans laquelle A représente un radical alkylène linéaire ou ramifié ayant de 1 à 6 atomes de carbone et R représente

- soit un radical alkyloxy, alkényloxy ou alkynyloxy ayant au plus 6 atomes de carbone,
- soit un radical alkylthio, alkénylthio ou alkynylthio ayant au plus 6 atomes de carbone, l'atome de soufre étant éventuellement oxydé en sulfoxyde ou en sulfone,
- soit un radical aryloxy, aralkyloxy,
- soit un radical arylthio, aralkylthio, l'atome de soufre étant éventuellement oxydé en sulfoxyde ou en sulfone, chacun de ces radicaux étant éventuellement substitué par un ou plusieurs des groupements suivants:
  hydroxy, alkoxy ou alkylthio ayant de 1 à 6 atomes de carbone, vinyloxy, allyloxy, éthynyloxy, propargyloxy, vinylthio, allylthio, éthynylthio, propargylthio, halogène, amino, méthylamino, diméthylamino, diéthylamino, 1-pipérazinyle, 4-méthyl pipérazin-1-yle, 1-pipéridinyle, 1-pyridinium, N-morpholino, 1-pyrrolyle, pyrrolidinyle, 1-imidazolyle, 1-pyridazinium, 1-pyrimidinium; les radicaux aryloxy, arylthio, aralkyloxy et aralkylthio étant de plus éventuellement substitués par les radicaux méthyle, éthyle, propyle, carbamoyle, aminométhyle, diméthylaminométhyle, aminoéthyle, diméthylaminoéthyle, carboxyle, méthyloxycarbonyle, éthyloxycarbonyle,
- soit R représente un radical

  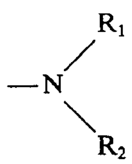

  dans lequel $R_1$ et $R_2$, identiques ou différents, représentent un atome d'hydrogène, un radical alkyle ayant de 1 à 6 atomes de carbone, ou $R_1$ et $R_2$ forment avec l'atome d'azote auquel ils sont liés un hétérocycle choisi dans le groupe formé par 1-pipéridinyle, 1-pipérazinyle, 4-méthyl pipérazin-1-yle, 1-pyridinium, N-morpholinyle, 1-pyrrolyle, 1-imidazolyle, 1-pyridazinium, 1-pyrimidinium,
- soit R représente un groupement ammonium quaternaire,
- soit R représente un atome d'halogène,
- soit R représente un groupement 1,2-époxyéthyle ou 2,2-diméthyl 1,2-époxyéthyle ou un radical résultant de l'ouverture de ce groupement par un réactif nucléophile,
- soit R représente un groupement

  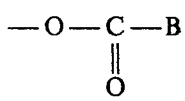

  dans lequel B représente soit un radical alkyle ou alkyloxy ayant au plus 6 atomes de carbone soit un radical aryle, aralkyle, aryloxy, ou aralkyloxy,
- soit R représente un radical formyle libre ou protégé, un radical carboxyle libre, estérifié ou salifié, un radical thiocyanate, un radical nitrile, acyle ou un radical carbamoyle, Ra représente un atome d'hydrogène ou un radical acyle; le trait ondulé signifie que les produits peuvent se trouver sous la forme syn ou anti ou sous la forme d'un mélange syn et anti;

ainsi que les sels d'addition de ces produits avec les acides minéraux ou organiques.

Parmi les valeurs de A, on peut citer les radicaux alkylènes linéaires tels que méthylène, éthylène, triméthylène, tétraméthylène, pentaméthylène, hexaméthylène et les radicaux alkylènes ramifiés tels que:

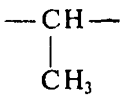 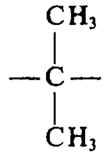 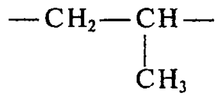 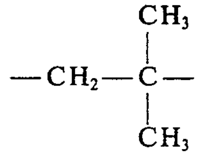

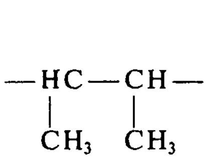 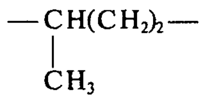 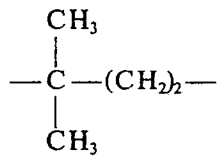 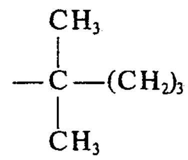

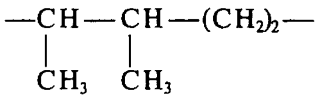

Parmi les valeurs de R on peut citer les valeurs mentionnées aux points a) à n) ci-dessous:

a) R = alkoxy ayant de 1 à 6 atomes de carbone: méthoxy, éthoxy, propyloxy, isopropyloxy, n-butyloxy, isobutyloxy, tert-butyloxy, n-pentyloxy, isopentyloxy, sec-pentyloxy, tert-pentyloxy, néopentyloxy, n-hexyloxy, sec-hexyloxy, tert-hexyloxy.

b) R = alkylthio: les mêmes valeurs peuvent être reprises en remplaçant l'atome d'oxygène par un atome de soufre, exemple: méthylthio, éthylthio ... De plus, l'atome de soufre peut être oxydé, exemple: méthylsulfinyle, méthylsulfonyle ...

c) R = alkényloxy ayant au plus 6 atomes de carbone: vinyloxy, 1-propényloxy, allyloxy, 1-butényloxy, 2-butényloxy, 3-butényloxy, 2-méthyl 1-butényloxy, penténylo xy, hexényloxy, 3-méthyl 2-butényloxy.

d) R = alkénylthio; les mêmes valeurs peuvent être reprises en remplaçant l'oxygène par un soufre éventuellement oxydé.

e) R = alkynyloxy ayant au plus 6 atomes de carbone: éthynyloxy, propargyloxy, propynyloxy, butynyloxy, pentynyloxy, hexynyloxy.

f) R = alkynylthio: les mêmes valeurs peuvent être reprises en remplaçant l'oxygène par un soufre éventuellement oxydé.

g) R = aryloxy: phényloxy, thiényloxy, furyloxy, thiazolyloxy, thiadiazolyloxy, oxazolyloxy, tétrazolyloxy, pyrrolyloxy, imidazolyloxy, pyrazolyloxy, isothiazolyloxy, isoxazolyloxy, triazolyloxy, thiatriazolyloxy, pyridyloxy, ainsi que les groupements condensés suivants: benzothiényloxy, benzofuryloxy, indolyloxy, benzimidazolyloxy.

h) Les groupements arylthio éventuellement oxydés correspondants peuvent bien entendu être utilisés, exemple: phénylthio, phénylsulfonyle, phénylsulfinyle ...

i) R = aralkyloxy: benzyloxy, phényléthyloxy, phénylpropyloxy, thiénylméthyloxy, thiényléthyloxy, thiénylpropyloxy, furfuryloxy, furyléthyloxy, furylpropyloxy, thiazolylméthyloxy, thiazolyléthyloxy, tétrazolylméthyloxy, thiadiazolylméthyloxy, thiadiazolyléthyloxy.

j) Les groupements aralkylthio éventuellement oxydés correspondants peuvent bien entendu être utilisés.

Les groupements que peut représenter R et qui sont indiqués dans les chapitres a) à j) ci-dessus peuvent être, en particulier substitués par un ou plusieurs des radicaux ou atomes suivants:

hydroxy, méthyloxy, éthyloxy, propyloxy, isopropyloxy, butyloxy, sec-butyloxy, tert-butyloxy, vinyloxy, allyloxy, éthynyloxy, propargyloxy, méthylthio, éthylthio, propylthio, isopropylthio, butylthio, sec-butylthio, tert-butylthio, vinylthio, allylthio, éthynylthio, propargylthio, fluoro, chloro, bromo, iodo, amino, méthylamino, diméthylamino, diéthylamino, 1-pipérazinyle, 4-méthyl pipérazin-1-yle, 1-pipéridinyle, 1-pyridinium, N-morpholino, 1-pyrrolyle, pyrrolidinyle, 1-imidazolyle, 1-pyridazinium, 1-pyrimidinium.

Les substituants indiqués dans les chapitres g) à j) peuvent de surcroit être substitués par les radicaux: méthyle, éthyle, propyle, carbamoyle, aminométhyle, diméthylaminométhyle, aminoéthyle, diméthylaminoéthyle, carboxyle, méthyloxycarbonyle, éthyloxycarbonyle.

k) le radical

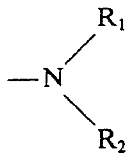

que peut représenter R, peut être un radical amino, méthylamino, diméthylamino, hydroxyéthylamino, dihydroxyéthylamino, 1-pipéridinyle, 1-pyridinium, N-morpholinyle, 1-pyrrolyle, 1-imidazolyle, 1-pyridazinium, 1-pyrimidinium.

l) R peut également représenter un radical triméthylammonium, ainsi que les ammoniums quaternaires dérivés des radicaux hétérocycliques tels que 1-pyridinium cités au paragraphe k).

m) R peut également représenter un radical fluoro, chloro, bromo ou iodo.

n) R peut représenter un radical 1-2-époxyéthyle

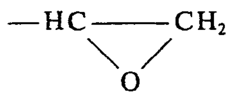

éventuellement substitué, tel que

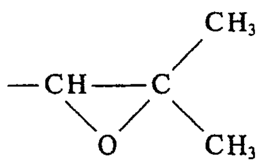

ou un radical résultant de l'ouverture de la fonction époxyde par un nucléophile.

Comme exemple de tels radicaux on peut citer les radicaux obtenus par action des amines, des sulfures, des phénates, des alcoolates, ou encore d'un acide halohydrique tel que l'acide fluorhydrique.

On obtient alors, par exemple, les structures suivantes:

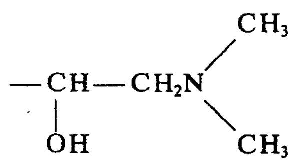

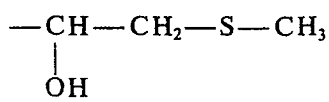

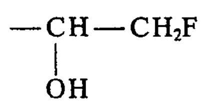

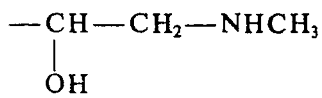

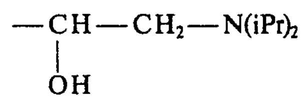

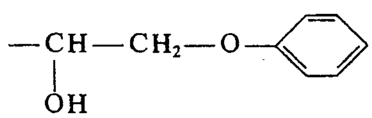

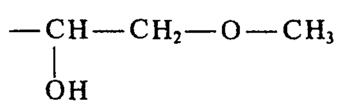

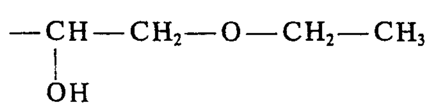

Les valeurs de B peuvent être choisies dans les valeurs indiquées ci-dessus pour R lorsque R représente un radical alkyloxy, aryloxy, ou aralkyloxy éventuellement substitué.

Les valeurs alkyle, aryle, aralkyle éventuellement substituées peuvent être les valeurs correspondantes telles que méthyle, éthyle, propyle . . . phényle, benzyle, etc.

Parmi les radicaux formyle protégés, on peut citer plus spécialement les radicaux du type acétal. On préfère les radicaux suivants: 1,3-dioxolan-2-yle, diméthoxyméthyle, diéthoxyméthyle.

Comme radicaux carboxyle estérifiés que peut représenter R on peut citer les radicaux alkoxycarbonyle ayant au plus 7 atomes de carbone tels que méthoxycarbonyle, éthoxycarbonyle, propyloxycarbonyle, isopropyloxycarbonyle, butyloxycarbonyle.

On peut également citer les radicaux alkoxy alkoxycarbonyle tels que méthoxyméthoxycarbonyle, isopropyloxyméthoxycarbonyle, les radicaux alkylthiométhoxycarbonyle tels que méthylthiométhoxycarbonyle, isopropylthiométhoxycarbonyle, les radicaux acyloxyalkoxycarbonyle tel que pivaloyloxyméthoxycarbonyle, acétoxyéthoxycarbonyle.

Parmi les sels formés avec le groupement carboxyle on peut citer les sels de sodium, potassium, lithium, calcium, magnésium, ammonium ou les sels formés avec les bases organiques aminées telles que la triméthylamine, la diéthylamine, la triéthylamine, le tris (hydroxyméthyl) aminométhane.

Parmi les radicaux acyle, on peut citer notamment les radicaux acétyle, propionyle, butyryle, isobutyryle, n-valéryle, isovaléryle, tert-valéryle, pivalyle.

Parmi les valeurs de Ra on peut citer en particulier les radicaux alkanoyle tel que acétyle, propionyle, n-butyryle, isobutyryle, n-valéryle, isovaléryle, pivalyle, acryloyle.

Comme exemple de sels d'addition des présents dérivés avec les acides minéraux ou organiques, on peut citer les sels formés avec les acides acétique, propionique, trifluoroacétique, maleique, tartrique, méthanesulfonique, benzènesulfonique, p-toluènesulfonique, chlorhydrique, bromhydrique, iodhydrique, sulfurique, phosphorique et spécialement les acides stéarique, éthylsuccinique ou laurylsulfurique.

La présente invention concerne notamment les produits tels que définis ci-dessus dans lesquels R représente un groupement alkyloxy, alkényloxy, alkynyloxy, alkylthio, alkénylthio, alkynylthio, aryloxy, aralkyloxy, arylthio ou aralkylthio éventuellement substitué par un ou plusieurs des groupements suivants:

- alkyloxy ou alkylthio ayant de 1 à 6 atomes de carbone,
- halogène,

ou R représente le groupement

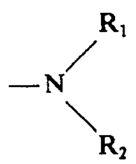

dans lequel $R_1$ et $R_2$ ont la signification indiquée précédemment.

La présente invention concerne plus spécialement les produits tels que définis précédemment et dans lesquels R représente

soit un radical alkyloxy éventuellement substitué par un radical alkyloxy ou par un radical dialkylamino;
soit un radical aryloxy ou aralkyloxy éventuellement substitué par un atome d'halogène;
soit un radical alkylthio ou arylthio éventuellement substitué par un atome d'halogène;
soit un radical dialkylamino;
soit un atome d'halogène.

La présente invention est tout spécialement dirigée vers les produits de formule (I')

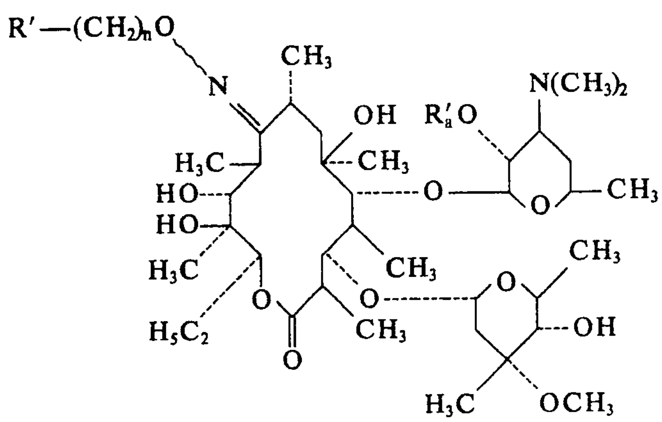

(I')

dans laquelle n représente un entier de 1 à 6 et R' représente

- soit un radical alkyloxy ou alkyloxyalkyloxy ayant au plus 6 atomes de carbone,
- soit un radical phényloxy ou benzyloxy éventuellement substitué par un atome de chlore,
- soit un radical dialkylamino;

R' a représente un atome d'hydrogène ou un radical alkanoyle ayant de 2 à 6 atomes de carbone, ainsi que leurs sels d'addition avec les acides minéraux ou organiques; produits de formule (I') correspondant aux produits de formule (I) dans laquelle A représente un radical alkylène saturé $-(CH_2)_n-$, R a les valeurs de R' et Ra a les valeurs de R' a et parmi ces produits ceux dans lesquels R' représente

- soit un radical

$$CH_3—(CH_2)_{n1}—O—$$

dans lequel n1 représente un entier de 0 à 3,

- soit un radical

$$CH_3—(CH_2)_{n1}—O—(CH_2)_{n2}—O—$$

dans lequel n1 a la signification précédente et n2 représente un entier de 1 à 3;

et ceux dans lesquels
R' représente un radical

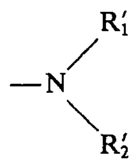

dans lequel $R'_1$ et $R'_2$ représentent un radical alkyle ayant de 1 à 3 atomes de carbone.

Parmi les produits décrits en exemples, on peut citer tout particulièrement les produits suivants:

Le 9-[O[(2-méthoxyéthoxy) méthyl]oxime] de l'érythromycine, ainsi que les sels d'addition de ce produit avec les acides minéraux ou organiques,
le 9-[O-[2-(diméthylamino) éthyl] oxime] de l'érythromycine, ainsi que les sels d'addition de ce produit avec les acides minéraux ou organiques, et
le 9-[O[2-(diéthylamino) éthyl]oxime] de l'érythromycine, ainsi que les sels d'addition de ce produit avec les acides minéraux et organiques.

La présente invention a également pour objet un procédé de préparation des produits de formule (I) telle que définie ci-dessus, caractérisé en ce que
soit l'on traite, éventuellement en présence d'une base l'érythromycine de formule (II)

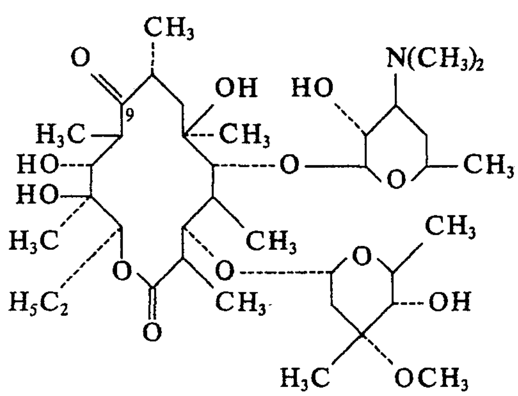

(II)

par un produit de formule

$$H_2N—O—A—R \quad (III)$$

dans lequel A et R sont définis comme précédemment ou par un sel d'acide fort de ce produit, pour obtenir un produit de formule ($I_1$) correspondant à un produit de formule I dans laquelle Ra représente un atome d'hydrogène,
soit l'on traite, éventuellement en présence d'une base, la 9-oxime d'érythromycine de formule (IV):

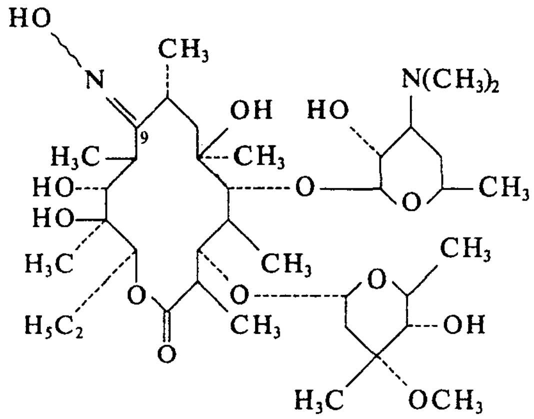

(IV)

sous l'une ou l'autre de ces formes isomères, ou sous forme de mélange, par un produit de formule

$$Hal—A—R \quad (V)$$

dans lequel A et R sont définis précédemment et Hal représente un atome d'halogène, pour obtenir un produit de formule ($I_1$) correspondant à un produit de formule (I) dans laquelle Ra représente un atome d'hydrogène, produit de formule ($I_1$) que, le cas échéant,

— dans le cas ou R représente un atome d'halogène ou un radical alkoxy, alkényloxy, alkynyloxy, alkylthio, alkénylthio ou alkynylthio substitué par un atome d'halogène, l'on peut également traiter par un réactif de formule

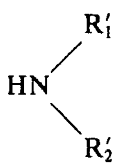

dans lequel

$R'_1$ et $R'_2$, identiques ou différents, représentent un atome d'hydrogène ou un radical ou un radical alkyle linéaire ou ramifié renfermant de 1 à 6 atomes de carbone ou forment avec l'atome d'azote un hétérocycle choisi dans le groupe formé par 1-pipéridinyle, 1-pipérazinyle, 4-méthyl pipérazin-1-yle, 1-pyridinium, N-morpholinyle, 1-pyrrolyle, 1-imidazolyle, 1-pyridazinium, 1-pyrimidinium, pour obtenir un produit correspondant de formule ($I_2$), correspondant à un produit de formule (I) dans laquelle Ra représente un atome d'hydrogène et R représente un radical

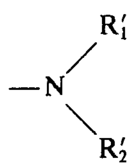

ou un radical alkoxy, alkényloxy, alkynyloxy, alkylthio, alkénylthio ou alkynylthio substitué par un radical

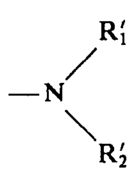

le radical

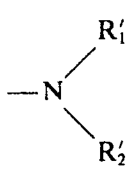

étant tel que défini précédemment;

— dans le cas où R représente un radical 1,2-époxyéthyle éventuellement substitué, l'on peut également traiter par un réactif nucléophile tel que défini précédemment, pour obtenir un produit correspondant de formule ($I_3$), correspondant à un produit de formule (I) dans laquelle Ra représente un atome d'hydrogène et R représente un radical résultant de l'ouverture de l'époxy;
produits de formule (I) dans laquelle Ra représente un atome d'hydrogène que, le cas échéant, l'on estérifie pour obtenir des produits de formule (I) dans laquelle Ra représente un radical acyle, et produits de formule (I) que, si désiré, l'on salifie.

Dans un mode préférentiel d'exécution du procédé ci-dessus, le produit de formule (III) est utilisé sous forme de sel en particulier d'halohydrate, de préférence de chlorhydrate ou de bromhydrate.

La réaction est alors effectuée, de préférence, dans un milieu tamponné, par exemple en présence d'un excès d'une base organique aminée telle que la triéthylamine ou d'un mélange acide organique — sel alcalin de cet acide tel qu'un mélange acide acétique acétate de sodium.

On peut également opérer en présence de carbonate ou de carbonate acide de sodium ou de potassium ou de carbonate de calcium ou de baryum par exemple.

On opère de préférence dans un solvant alcoolique tel que le méthanol ou l'éthanol et de préférence en milieu anhydre.

La réaction du produit de formule (V) sur le produit de formule (IV) est effectuée de préférence, en présence d'une base telle que la triéthylamine ou en présence de carbonate ou de carbonate acide de sodium ou de potassium ou de carbonate de calcium ou de baryum.

On peut également utiliser un hydrure tel que l'hydrure de sodium.

On opère de préférence dans un solvant polaire tel que l'acétone, le diméthylformamide; le diméthylsulfoxyde ou l'hexaméthylphosphotriamide. On peut cependant utiliser un éther tel que l'éther éthylique, le tétrahydrofuranne ou le dioxanne.

Dans les deux cas précédents les réactions peuvent être conduites à des températures comprises entre la température ambiante et la température de reflux du solvant. La réaction peut être prolongée plusieurs heures voire plusieurs jours pour obtenir une transformation complète.

La salification et l'estérification éventuelles sont effectuées selon les méthodes usuelles.

L'invention a plus particulièrement pour objet un procédé de préparation des produits de formule (I) caractérisé en ce que l'on traite un produit de formule (II) par un chlorhydrate ou un bromhydrate d'un produit de formule (III) et en ce que l'on opère en présence d'une base organique aminée ou d'un carbonate de métal alcalino-terreux, ainsi qu'un procédé caractérisé en ce que l'on traite un produit de formule (IV) par un produit de formule (V) en présence d'une base choisie dans le groupe formé par le carbonate et le carbonate acide de sodium ou de potassium, le carbonate de calcium, le carbonate de baryum et l'hydrure de sodium.

Les produits de formule générale (I) possèdent une très bonne activité antibiotique sur les bactéries gram ⊕ telles que les staphylocoques, les streptocoques, les pneumocoques.

Ces propriétés rendent aptes lesdits produits pharmaceutiquement acceptables à être utilisés comme médicaments dans le traitement des affections à germes sensibles et, notamment, dans celui des staphylococcies, telles que septicémies à staphylocoques, staphylococcies malignes de la face ou cutanée, pyodermites, plaies septiques ou suppurantes, furoncles, anthrax, phlegmons, érésipèles, staphylococcies aigües primitives ou post grippales, bronchopneumonies, suppuration pulmonaires, les streptococcies telles que les angines aigües, les otites, les sinusites, la scarlatine, les pneumococcies telles que les pneumonies, les bronchites; la brucellose, la diphtérie, la gonococcie. Les produits de la présente invention sont également actifs contre des infections dues à des germes comme Haemophilus influenzae, Haemophilus Pertussis, Rickettsies, Mycoplasma pneumoniae.

La présente invention a donc également pour objet, à titre de médicaments et, notamment, de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment, de médicaments antibiotiques, les produits préférés de formule (I) définis précédemment et leurs sels, pharmaceutiquement acceptables.

Parmi les produits décrits en exemple, les produits suivants constituent des médicaments préférés:
le 9-[O[(2-méthoxyéthoxy) méthyl]oxime] de l'érythromycine ainsi que les sels d'addition de ce produit avec les acides minéraux ou organiques pharmaceutiquement acceptables;
le 9-[O-[2-(diméthylamino)éthyl]oxime] de l'érythromycine ainsi que les sels d'addition de ce produit avec les acides minéraux ou organiques, pharmaceutiquement acceptables et
le 9-[O-[2-(diéthylamino) éthyl]oxime] de l'erythromycine, ainsi que les sels d'addition de ce produit avec les acides minéraux ou organiques pharmaceutiquement acceptables.

L'invention s'étend aux compositions pharmaceutiques renfermant comme principe actif au moins un des médicaments définis ci-dessus.

Ces compositions peuvent être administrées par voie buccale, rectale, parentérale, ou par voie locale en application topique sur la peau et les muqueuses.

La voie préférée est la voie buccale.

Elles peuvent être solides ou liquides et se présenter sous les formes pharmaceutiques couramment utilisées en médecine humaine, comme par exemple, les comprisés, simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la

gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 0,250 g et 4 g par jour par voie orale, chez l'homme, avec le produit décrit à l'exemple 1 ou 5.

Les produits objet de la présente demande présentent, in vitro, des propriétés voisine de celles de l'érythromycine.

In vivo les produits de la présente demande présentent deux avantages très intéressants:
– d'une part, la protection de l'animal infecté expérimentalement est réalisée à des doses inférieures à celles de l'érythromycine ou du propionate d'érythromycine,
– d'autre part, les résultats de la pharmacocinétique mettent en évidence une nette supériorité par rapport à l'érythromycine.

Les produits de formule (III) peuvent par exemple être préparés selon le procédé général décrit dans Angew. Chem. 68, 1956 No 8 p. 303.

L'oxime d'érythromycine de formule (IV) est décrite dans le brevet britannique 1 100 504.

Les produits de formule (V) sont connus ou peuvent être préparés selon des techniques connues.

En plus des exemples suivants qui illustrent l'invention sans toutefois la limiter, on peut également mentionner, comme produits préférentiels ceux dont les substituants R et A ont les valeurs suivantes:

| A | R | Ra |
|---|---|---|
| $—CH_2—$ | $CH_3—(CH_2)_3—O—$ | H |
| $—CH_2—$ | iPr—O— | H |
| $—CH_2—$ | 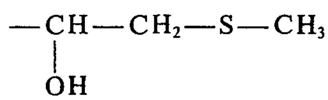 | H |
| $—CH_2—$ | 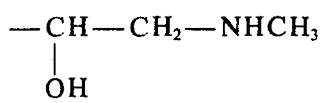 | H |
| $—(CH_2)_2—$ | $CH_3—O—$ | H |
| $—(CH_2)_2—$ | Br | H |
| $—(CH_2)_3—$ | 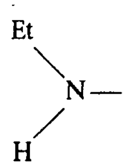 | H |
| $—(CH_2)_3—$ | 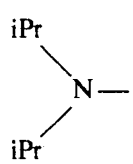 | H |
| $—(CH_2)_2—$ | $—NHCH_3$ | H |
| $—(CH_2)_2—$ | 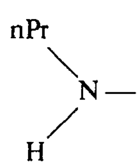 | H |
| $—(CH_2)_3—$ | $H_2N—$ | H |

En plus des produits dérivés de l'érythromycine A qui font l'objet de la présente demande, il est évident que les dérivés correspondants des érythromycines B et C peuvent être préparés. Dans les produits dérivés de l'érythromycine B, le groupement hydroxyle en position 12 est remplacé par un hydrogène, dans ceux dérivés de l'érythromycine C le groupement méthoxy en position 3" est remplacé par un hydroxyle.

## Exemple 1

### 9-[O-(2-méthoxyéthoxy) méthyloxime] de l'érythromycine

On dissout 1,87 g d'oxime de l'érythromycine dans 25 $cm^3$ d'acétone, ajoute 0,94 g de bicarbonate de sodium et 0,3 $cm^3$ de chlorure de [(méthoxy)éthoxy]méthyle, puis porte au reflux sous atmosphère inerte pendant 16 heures en ajoutant une fois 0,3 $cm^3$ de chlorure de [(méthoxy)éthoxy]méthyle. On essore l'insoluble, concentre le filtrat au ⅓ de son volume, ajoute une solution de bicarbonate de sodium et extrait à l'éther éthylique, sèche et concentre à sec. Le résidu est chromatographié sur silice. On élue par un mélange benzène-triéthylamine (15—1) et obtient 1,29 g de produit attendu.

$\alpha_D$ = −77,5° ± 2° (concentration: 0,45 % chloroforme)
Rf = 0,2 (benzène-triéthylamine 9—1 support = silice)

Analyse: $C_{41}H_{76}N_2O_{15}$ = 837

| | | | |
|---|---|---|---|
| Calculé: | C% 58,8 | H% 9,16 | N% 3,35 |
| Trouvé: | C% 59 | H% 9,20 | N% 3,30 |

## Exemple 2

### 9-[O-[2-(4-chloro-phénoxy) éthyl] oxime] de l'érythromycine

On dissout 11,4 g de (para-chloro phénoxy) 2-éthoxyamine dans 230 $cm^3$ de méthanol anhydre, ajoute 18,5 $cm^3$ de méthanol chlorhydrique 3,25 N et agite pendant 30 minutes à température ambiante. On ajoute 21 g d'érythromycine, 11 g de carbonate de baryum et 35 $cm^3$ de méthanol et agite pendant 18 heures à température ambiante. On filtre l'insoluble, verse le filtrat dans 750 $cm^3$ d'eau contenant 75 $cm^3$ d'ammoniaque concentrée, extrait au chloroforme, lave avec une solution d'ammoniaque diluée à 10%, sèche, concentre et obtient 35 g de résine.

Par chromatographie sur silice en éluant par un mélange chloroforme-triéthylamine (9/1) on obtient 1,925 g de produit attendu et 2,03 g de produit impur que l'on purifie par chromatographie sur silice à l'aide du mélange benzène-triéthylamine (15—1). On obtient alors 1,42 g de produit attendu.

$[\alpha]_D$ = 79,5° ± 2,5° (c = 1% éthanol)

## Exemple 3

### 9-[O-(2-éthoxyéthyl)oxime] de l'érythromycine

On dissout 3,7 g d'érythromycine dans 25 $cm^3$ de méthanol sec, ajoute 1,4 $cm^3$ de triéthylamine puis 2,8 g de chlorhydrate de O-(2-éthoxy éthyl) hydroxylamine. On agite le mélange pendant 96 heures à température ambiante sous atmosphère inerte, puis le verse dans l'ammoniaque diluée (12 $cm^3$ d'ammoniaque concentrée à 28% dans 50 $cm^3$ d'eau) et refroidit dans l'eau glacée. Le précipité obtenu est lavé à l'eau, repris au chlorure de méthylène. On lave la phase organique avec une solution aqueuse saturée de chlorure de sodium, sèche, concentre à sec, et obtient 3,83 g de produit brut que l'on purifie par chromatographie sur silice, élue par un mélange benzène-triéthylamine (15—1) pour isoler 1,9 g de produit attendu.

$[\alpha]_D$ = −73° ± 3° (C = 0,6% $CHCl_3$)

## Exemple 4

### 9-[O-[2-éthoxy éthoxyméthyl]oxime] de l'érythromycine

On dissout 1,89 g d'oxime de l'érythromycine dans 25 $cm^3$ d'acétone, ajoute 1 g de bicarbonate de sodium et obtient 0,36 $cm^3$ de 1-(chlorométhoxy) 2-éthoxy éthane puis agite pendant 24 heures à

température ambiante et au reflux pendant 75 heures en ajoutant une fois 0,2 $cm^3$ puis une deuxième fois 0,15 $cm^3$ de 1-(chlorométhoxy) 2-éthoxy éthane. On filtre l'insoluble, verse le filtrat dans 100 $cm^3$ d'eau et extrait à l'éther éthylique, sèche, concentre à sec sous pression réduite et obtient 2,5 g de produit brut. On chromatographie ce dernier sur silice en éluant avec un mélange benzène-triéthylamine (9—1) et recueille 1,72 g de produit attendu.

$[\alpha]_D = -79° \pm 2°$ (C = 0,7% éthanol)

## Exemple 5

### 9-[O-[2-(diméthylamino) éthyl] oxime] de l'érythromycine

On dissout 5,6 g d'oxime d'érythromycine dans 80 $cm^3$ d'acétone, ajoute 6,3 g de bicarbonate de sodium et 1,65 g de chlorhydrate de diméthylamino chloroéthane et porte au reflux pendant 4 jours et demi, en ajoutant en 4 fois 3,3 g de chlorhydrate de diméthylamino chloréthane. On filtre l'insoluble, concentre le filtrat qui cristallise pour donner 4,5 g de produit. On verse les eaux-mères dans 100 $cm^3$ d'eau et extrait à l'éther éthylique. On lave la phase éthérée avec une solution aqueuse saturée de chlorure de sodium, sèche et concentre à sec. Le résidu et le produit cristallisé obtenu ci-dessus sont chromatographiés sur silice. On élue avec un mélange chloroforme-triéthylamine (100—15) et obtient 4,38 g de produit attendu.

$[\alpha]_D = -99,5° \pm 2°$ (1% éthanol).

## Exemple 6

### 9-[O-(2-méthoxyéthoxy) méthyl oxime] de l'érythromycine

On dissout 99,6 g d'oxime de l'érythromycine dans 1000 $cm^3$ d'acétone séchée. Après addition de 56 g de bicarbonate de sodium sec et de 15,2 $cm^3$ de chlorure de [(méthoxy)éthoxy] méthyle, on porte au reflux 15 heures en agitant et en plaçant sous atmosphère d'azote. On ajoute ensuite 7,6 $cm^3$ de chlorure de [(méthoxy)éthoxy] méthyle et porte de nouveau 8 heures au reflux. Enfin une troisième addition de 5 $cm^3$ du même produit a été suivie de 15 heures de reflux.

Le mélange est alors refroidi, l'insoluble est filtré et rincé à l'acétone.

On évapore le filtrat acétonique et obtient une résine que l'on triture dans 500 $cm^3$ d'une solution concentrée de bicarbonate de sodium.

Le produit amorphe obtenu est filtré, rincé à l'eau et séché à l'étuve. On répète trois fois cette préparation. On obtient au total 335,6 g de produit brut soit un rendement quantitatif.

Les 335,6 g de produit sont dissous dans 1,5 litre d'acétone pure. On ajoute 15 g de charbon actif et 15 g de Kieselgur.

On agite quelques minutes puis filtre, rince et lave par environ 300 $cm^3$ d'acétone. On répète le même traitement avec 3 g de charbon actif et 15 g de Kieselgur.

Après filtration on obtient une solution limpide. On lave, rince par 300 $cm^3$ d'acétone. Le filtrat est concentré à 1,5 litre environ sous vide.

On ajoute à chaud 800 $cm^3$ d'eau distillée puis amorce la cristallisation. On laisse la cristallisation se développer à 20—25°C jusqu'à prise en masse avant de refroidir à 0 + 5°C.

Les cristaux sont filtrés puis lavés par de l'acétone à 40% d'eau glacée.

On obtient un premier jet de 134,5 g et un deuxième jet de 75,9 g, enfin un troisième jet de 15,3 g soit un total de 225,7 g.

On recristallise 215 g de produit ci-dessus en les dissolvant dans 645 $cm^3$ d'acétone.

On rajoute 5 g de Kieselguhr.

On agite quelques minutes puis filtre. On obtient une solution limpide et peu colorée. On ajoute peu à peu 525 $cm^3$ d'eau distillée. On amorce la cristallisation au moyen de germes et par grattage. On laisse la cristallisation se développer à 20—25°C. Après deux heures il y a prise en masse. On refroidit à 0—5°C et y laisse une nuit.

On filtre les cristaux. On rince et lave par 100 $cm^3$ d'acétone à 40% d'eau. On sèche à 40°C sous vide.

On obtient 170 g de produit.

Un deuxième jet de 16 g puis un troisième jet de 9,5 g cristallisent dans les liqueurs mères.

On obtient en tout 195,5 g de produit pur.

Exemple 7

9-[O-(phénylméthoxy) méthyloxime] de l'érythromycine

On dissout 1,9 g d'oxime de l'érythromycine dans 25 $cm^3$ d'acétone, ajoute 1,2 g de bicarbonate de sodium et 0,53 $cm^3$ de chlorométhyl benzyléther. On agite d'abord 1 h à température ambiante sous azote. On porte la suspension au reflux dans l'acétone à 60°C pendant 25 heures. On ajoute 0,25 $cm^3$ de chlorométhyl benzyléther, au bout de 41 heures 30 on rajoute la même quantité et on arrête la réaction au bout de 45 heures.

On filtre l'insoluble et le rince à l'acétone. On verse dans 100 $cm^3$ d'eau et obtient une solution laiteuse. On extrait trois fois par 200 $cm^3$ d'éther éthylique. On lave les phases éthérées avec une solution saturée de chlorure de sodium. On sèche, évapore à sec sous pression réduite et obtient 3,4 g de résine que l'on purifie comme suit:

On chromatographie sur silice avec un éluant constitué de benzène-triéthylamine (9—1). On isole 1,2 g de produit purifié. Ce produit est dissout dans 20 $cm^3$ d'acétone puis on concentre jusqu'à un volume de 2 $cm^3$. On reprend à froid par 60 $cm^3$ d'éther de pétrole (Eb = 64—75°C). Le produit cristallise, on le filtre et le rince à l'éther de pétrole. On obtient 0,715 g de produit.

On redissout ce produit dans environ 100 $cm^3$ d'acétone et agite à environ 50°C avec du charbon actif pendant 10 minutes. On filtre à chaud.

On concentre la solution acétonique jusqu'à 2 $cm^3$ puis reprend par 100 $cm^3$ d'éther de pétrole (Eb = 64—75°C). Le produit cristallise de nouveau. On filtre les cristaux, rince à l'éther de pétrole et sèche sous vide à 60°C.

On obtient 0,585 g de produit pur.

$[\alpha]_D = -74,5° \pm 3°$ (0,25% éthanol).

Analyse: $C_{45}H_{76}N_2O_{14} = 869,1$

| | | | |
|---|---|---|---|
| Calculé: | C% 52,19 | H% 8,81 | N% 3,22 |
| Trouvé: | C% 62,19 | H% 8,8 | N% 3,1 |

Exemple 8

9-[O-[2-(diméthylamino) éthyl]oxime] de l'érythromycine

On dissout 11,2 g d'oxime d'érythromycine dans 160 $cm^3$ d'acétone puis introduit 3,6 g de carbonate de sodium puis ajoute 3,24 g de chlorhydrate de diméthylaminochloréthane. On agite au reflux pendant 93 heures. Après refroidissement, on filtre l'insoluble, rince à l'acétone. On évapore sous pression réduite une partie de l'acétone jusqu'à un volume de 150 $cm^3$. Le produit cristallise. On laisse à +5°C puis filtre les cristaux que l'on rince. On obtient 2,45 g de cristaux = fraction A. On reprend l'insoluble avec 200 $cm^3$ d'acétone et agite une demi-heure. On filtre, évapore les deux tiers de l'acétone. Du produit cristallise, on obtient 0,864 g de produit = fraction B. L'opération est répétée plusieurs fois et on obtient une fraction C = 3,37 g. On reprend de nouveau l'insoluble par environ 200 $cm^3$ d'acétone puis chauffe à 50°C en agitant une demi-heure. On filtre à chaud, évapore les deux tiers de l'acétone et refroidit. On filtre les cristaux, rince à l'acétone froid, puis sèche. On obtient une fraction D = 2,64 g. On récupère encore 0,661 g de produit à partir des eaux mères de la fraction A, soit en tout 9,989 g de cristaux. On recristallise les produits comme suit:

Les 9,989 g sont dissous dans environ 800 $cm^3$ d'acétone en présence de 1 g de charbon actif. On agite à 50°C pendant 15 minutes et filtre. On obtient une solution limpide et incolore. On concentre jusqu'à 250 $cm^3$ environ. Le produit cristallise. On refroidit, filtre les cristaux, les lave, sèche et obtient un premier jet de 8,27 g puis un deuxième jet de 0,714 g de produit attendu.

$[\alpha]_D = -86° \pm 2°$ à 1% dans le chloroforme.

Le produit est identique au produit de l'exemple 5.

Exemple 9

9-[O-[2-(diméthylamino) éthyl]oxime] de l'érythromycine

On introduit sous argon 40 $cm^3$ d'un mélange hexaméthylphosphotriamide-éther éthylique (3—1) et 2,247 g d'oxime d'érythromycine, puis en plusieurs fois 0,159 g d'hydrure de sosium en suspension à 50% dans l'huile. On agite 15 minutes, puis introduit goutte à goutte une solution de 21 $cm^3$ d'hexaméthylphosphotriamide, 0,518 g de chlorhydrate de chlorure de diméthylaminoéthyle et 0,173 g

d'hydrure de sodium et agite 20 minutes. La réaction est terminée au bout de 4 heures. On verse dans 30 $cm^3$ d'eau saturée par 60 g d'acétate d'ammonium, extrait à l'éther, lave à l'eau, sèche, évapore à sec. On dilue à l'eau, extrait au toluène, sèche, évapore sous pression réduite, reprend avec un minimum de pentane, amorce avec du produit précédemment obtenu, agite 3 heures à température ambiante, essore et sèche à 60°C. On obtient 0,21 g de produit attendu F = 233°C. Les différentes liqueurs mères n'ont pas été traitées. Le produit est identique aux produits obtenus dans les exemples 5 et 8.

## Exemple 10

### 9-[O-[2-(diméthylamino) éthyl]oxime] de l'érythromycine

On remplace, dans le mode opératoire décrit à l'exemple 9, le mélange hexaméthylphosphotriamide-éther par du diméthylformamide. On place 0,75 g d'oxime d'érythromycine dans 3,5 $cm^3$ de diméthylformamide. On ajoute 53 mg d'hydrure de sodium au demi. On agite 15 minutes et ajoute 0,158 g de chlorhydrate de chlorure de diméthylaminoéthyle et 53 g d'hydrure de sodium au demi. Au bout de 4 heures à température ambiante. On ajoute 29 mg de chlorhydrate de chlorure de diméthylaminoéthyle et 11 mg d'hydrure de sodium au demi. On agite une nuit, sature au gaz carbonique, verse dans 35 $cm^3$ d'eau, essore, rince, reprend au chloroforme, sèche et évapore. Le résidu est cristallisé dans 2 $cm^3$ de pentane. On obtient 0,428 g de produit attendu. Après reprise des liqueurs mères, on obtient un deuxième jet de 210 mg de produit identique aux produits obtenus aux exemples 5, 8 et 9.

## Exemple 11

### 9-[O-[(4-chlorophénoxy)méthyl]oxime] de l'érythromycine

Dans 60 $cm^3$ d'éther éthylique, on introduit 2,247 g d'oxime d'érythromycine et ajoute en plusieurs fois 0,159 g d'hydrure de sodium en suspension à 50% dans l'huile. On agite 15 minutes supplémentaires puis ajoute 6 $cm^3$ d'hexaméthylphosphotriamide. On introduit ensuite goutte à goutte en 15 minutes 6 $cm^3$ d'une solution éthérée contenant 0,45 $cm^3$ de chlorure de p-chloro anisole. La réaction est complète en 40 minutes. On verse dans 30 $cm^3$ d'eau saturée par 60 g d'acétate d'ammonium, extrait par 3 fois 15 $cm^3$ d'éther, lave à l'eau. Un précipité apparait. On étend à l'acétate d'éthyle jusqu'à dissolution totale, décante, lave à l'eau puis par une solution saturée au chlorure de sodium, sèche, évapore le solvant, reprend au pentane et agite 16 heures à température ambiante. On essore, rince au pentane, sèche et obtient 1,99 g de produit attendu. Le produit est purifié comme suit:

On agite 1,89 g de produit avec 3,8 g de silicate de magnésium activé dans 40 $cm^3$ de benzène pendant une heure, essore, rince avec 5 $cm^3$ de benzène et évapore à sec. On renouvelle l'opération. On reprend par 5 $cm^3$ de pentane et agite une nuit à température ambiante. On essore, rince avec 0,5 $cm^3$ de pentane, sèche et obtient 0,903 g de produit attendu.

F = 90°C.
$[\alpha]_D$ = $-64,5° \pm 2,5°$ (c = 0,5% éthanol).
RMN: $CDCl_3$ ppm

2,27 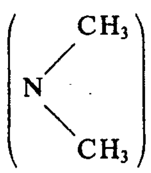

5,65 (d, d, 7 hz) $-N\sim OCH_2O$

Analyse

| | | | | |
|---|---|---|---|---|
| Calculé: | C% 59,41 | H% 8,27 | N% 3,15 | Cl% 3,98 |
| Trouvé: | C% 59,5 | H% 8,4 | N% 3,0 | Cl% 4,0 |

## Exemple 12

### 9-O(cyanométhyl) oxime de l'érythromycine

On introduit sous argon 80 $cm^3$ d'acétone et 3 g d'oxime d'érythromycine et ajoute en plusieurs fois 0,21 g d'hydrure de sodium en suspension dans l'huile, on agite 15 minutes puis rajoute 0,384 g de chlorure d'acétonitrile en solution dans 1 $cm^3$ d'acétone. Au bout de 20 minutes, on rajoute 0,141 g

d'hydrure de sodium, puis 10 minutes après 0,256 g de chlorure d'acétonitrile. Après 10 minutes, on rajoute 70 mg d'hydrure de sodium et 0,125 g de chlorure d'acétonitrile. On sature au gaz carbonique pendant une heure et demie, évapore à sec sous pression réduite, sèche. On chromatographie sur silice en éluant avec un mélange éther-triéthylamine (85—15). On rassemble les fractions, évapore à sec, cristallise dans l'éther de pétrole (Eb: 65—75°C). Après essorage, lavage et séchage, on obtient 1,8 g de produit attendu.

F = 140°C.
$[\alpha]_D$ = −74,5 ± 2,5° (c = 0,6% dans $CHCl_3$).

Analyse:

| | | | | |
|---|---|---|---|---|
| Calculé: | C% 59,45 | H% 8,83 | N% 5,33 | |
| Trouvé: | C% 59,2 | H% 9,0 | N% 5,0 | |

## Exemple 13

### 9-[O-(2-éthylthioéthyl) oxime] de l'érythromycine

1. On introduit sous argon 90 $cm^3$ de méthyléthylcétone et 10 $cm^3$ de 2-chloroéthyl éthyl sulfure et 12,9 g d'iodure de sodium. On porte au reflux pendant une heure, refroidit, essore jusqu'à élimination du chlorure de sodium formé. Le filtrat est conservé dans un flacon hermétiquement bouché.

2. On introduit sous argon 3,75 g d'oxime d'érythromycine dans 24 $cm^3$ d'une solution d'hexaméthylphosphotriamide-éther (1—1). On chasse l'éther sous pression réduite puis introduit 0,26 g d'hydrure de sodium en suspension dans l'huile. On poursuit l'agitation 15 minutes et ajoute en 20 minutes 50 $cm^3$ de la solution obtenue en 1.

On rajoute en plusieurs fois 0,6 g d'hydrure de sodium. Après 50 minutes, on rajoute 0,59 g d'hydrure de sodium. 60 minutes après, on verse le mélange réactionnel dans 240 $cm^3$ d'une solution aqueuse de chlorure de sodium saturée aux $^3/_4$ et contenant 10 g de carbonate acide de sodium. On agite 10 minutes à température ambiante, essore le précipité formé, le solubilise dans du chloroforme, le sèche, évapore à sec et obtient 5 g d'un liquide que l'on chromatographie sur silice (éluant toluène-triéthylamine 9—1). On réunit les fractions homogènes et les évapore à sec. On obtient 1,1 g d'une résine que l'on triture dans de l'éther de pétrole (Eb: 65—75°C). On sèche et obtient 1,036 g de produit attendu.

F = 110°C.
$[\alpha]_D$ = 72,5° ± 2° (c = 1% dans $CHCl_3$).

Analyse:

| | | | | |
|---|---|---|---|---|
| Calculé: | C% 58,83 | H% 9,15 | N% 3,35 | S% 3,83 |
| Trouvé: | C% 58,8 | H% 9,3 | N% 3,3 | S% 3,7 |

## Exemple 14

### 9-[O-(2-cyano éthyl)oxime] de l'érythromycine

On introduit sous argon 10 $cm^3$ d'hexaméthylphosphotriamide et 3 g d'oxime de l'érythromycine. Après dissolution, on ajoute 0,2 g d'hydrure de sodium en suspension dans l'huile et goutte à goutte 0,4 $cm^3$ de bromopropionitrile purifié. Après 45 minutes, on rajoute par fraction environ 0,1 g d'hydrure de sodium et 4 gouttes de bromopropionitrile. On renouvelle l'opération en introduisant en plus 0,7 g d'hydrure de sodium et 0,42 $cm^3$ de bromopropionitrile. On verse le mélange dans une solution saturée aux $^3/_4$ de chlorure de sodium dans 240 $cm^3$ d'eau contenant 2,4 g de carbonate acide de sodium. Après une heure d'agitation, on essore sur filtre, rince à l'eau saturée de chlorure de sodium. On reprend le précipité par 200 $cm^3$ de chloroforme, agite 1 heure, puis essore l'insoluble, le lave au chloroforme. Le filtrat et les liqueurs de lavage sont réunis puis décantés. On sèche la fraction chloroformique, filtre évapore à sec sous pression réduite. On obtient une poudre amorphe soit 3,26 g de produit brut. 2 g de ce produit sont chromatographiés sur silice et élués par un mélange chloroforme-triéthylamine (95—5). La fraction homogène donne 1,348 g de résine que l'on reprend au pentane et agite 16 heures. On évapore à sec sous pression réduite, reprend avec 10 $cm^3$ de pentane, agite une demi-heure puis essore, rince et sèche. On obtient 0,766 g de cristaux blancs.

F = 210°C.
$[\alpha]_D^{20}$ = −77,5° ± 1,5° (c = 1% dans $CHCl_3$).

Analyse:
Calculé: C% 59,9 H% 8,92 N% 5,24
Trouvé: C% 59,6 H% 9,0 N% 5,5

## Exemple 15

### 9-[O-(3,3-diméthyl 2-oxo butyl) oxime] de l'érythromycine

On introduit sous argon 10 $cm^3$ d'hexaméthylphosphotriamide et ajoute en plusieurs fois 3 g d'oxime d'érythromycine. A la solution obtenue, on rajoute 0,22 g d'hydrure de sodium en suspension dans l'huile. On refroidit à 10°C et ajoute goutte à goutte 0,6 $cm^3$ de bromure de 3,3-diméthyl 2-oxo butyle. On maintient la réaction à 10—12°C pendant 25 minutes. On verse lentement dans 200 $cm^3$ d'une solution aqueuse de chlorure de sodium saturée aux $^3/_4$ et contenant 1,6 g de carbonate acide de sodium. On rince avec une solution aqueuse de chlorure de sodium saturée aux $^3/_4$. On agite 20 minutes, essore le précipité formé, le solubilise avec du chloroforme, décante la phase organique, la lave, sèche, évapore à sec sous pression réduite et obtient 3,28 g de produit impur. Les 3,28 g sont réunis avec 5,1 g de produit obtenu de la même façon. On chromatographie les 8,38 g de produit sur silice et élue avec un mélange toluène-chloroforme-triéthylamine (6-4-1). On réunit les fractions homogènes, évapore à sec sous pression réduite, reprend le résidu au pentane, agite, évapore à sec, agite dans 3 $cm^3$ de pentane, essore le précipité formé, le rince par 0,3 $cm^3$ de pentane. On sèche à poids constant et obtient 0,67 g d'une poudre blanche.

F = 146°C.
$[\alpha]_D^{20}$ = −63,5° ± 1,5° (c = 1% dans $CHCl_3$).

Analyse:
Calculé: C% 60,97 H% 9,28 N% 3,31
Trouvé: C% 60,8 H% 9,3 N% 3,2

## Exemple 16

### 9-[O-(3-éthylthio 2-hydroxypropyl)oxime] de l'érythromycine

On mélange 34 $cm^3$ d'un mélange éther-hexaméthylphosphotriamide (1—1) et 5 g d'oxime d'érythromycine. On chasse l'éther sous pression réduite, puis on introduit en plusieurs fois sous argon 0,352 g d'hydrure de sodium à 50% dans l'huile, agite 15 minutes, puis ajoute 1,25 g de chlorure de 3-éthylthio 2-hydroxypropyle. On rajoute après 15 minutes 0,352 g d'hydrure de sodium. Lorsque la réaction est terminée (on suit l'évolution de la réaction par chromatographie sur couche mince), on verse le mélange réactionnel dans 400 $cm^3$ d'une solution aqueuse saturée aux $^3/_4$ en chlorure de sodium et contenant 1,7 g de carbonate acide de sodium. On essore le précipité, le dissout dans le chloroforme, le sèche et évapore à sec sous pression réduite. On obtient 3,6 g d'une résine qui est réunie avec 3,7 g obtenus lors d'un essai identique et les chromatographie sur silice en éluant avec un mélange chloroforme-triéthylamine (9—1). On réunit les fractions homogènes, les évapore à sec et obtient 2,7 g de résine blanche. On la chromatographie de nouveau sur silice en éluant avec un mélange chloroforme-méthanol (1—1). On réunit les fractions contenant le produit attendu, les évapore à sec, obtient 1,116 g de résine blanche que l'on cristallise par agitation dans 20 $cm^3$ de pentane. On essore le précipité, le rince au pentane et sèche à poids constant. On obtient 0,93 g de produit attendu.

F = 142°C.
$[\alpha]_D^{20}$ = −76,5° ± 2,5° (c = 0,8% dans $CHCl_3$).

Analyse:
Calculé: C% 58,17 H% 9,07 N% 3,23 S% 3,7
Trouvé: C% 58,0 H% 9,1 N% 3,2 S% 3,6

## Exemple 17

### 9-[O-[2-(2-hydroxyéthoxy) éthyl] oxime] de l'érythromycine

On introduit sous argon 10 $cm^3$ d'hexaméthylphosphotriamide et 3 g d'oxime de l'érythromycine. On agite 25 minutes et ajoute en plusieurs fois 0,212 g d'hydrure de sodium à 50% dans l'huile et après 15 minutes d'agitation, ajoute 0,6 g de 2-(2-chloroéthoxy) éthanol. Après 15 minutes, on rajoute

0,212 g d'hydrure de sodium puis 0,6 g de 2-(2-chloroéthoxy) éthanol. Après 15 minutes, on rajoute 0,212 g d'hydrure de sodium puis 0,6 g de 2-(2-chloroéthoxy) éthanol. Après 1 heure, on arrête la réaction en versant lentement le mélange dans 240 $cm^3$ d'une solution saturée aux $^3/_4$ en chlorure de sodium et contenant 3 g de carbonate acide de sodium. On agite 10 minutes, essore le produit formé, le dissout dans le chloroforme, lave avec une solution aqueuse saturée de chlorure de sodium, sèche, évapore à sec sous pression réduite et obtient 3,5 g d'une résine que l'on chromatographie sur silice (éluant: toluène-triéthylamine 9—1). On évapore les fractions contenant le produit attendu, soit 2,9 g d'une résine que l'on chromatographie à nouveau sur silice (éluant: chloroforme-triéthylamine 12,5—7,5). On obtient 1,8 g de produit attendu que l'on purifie de la façon suivante: 1,063 g de résine sont triturés puis décantés 3 fois de suite avec 5 $cm^3$ d'une solution saturée de chlorure de sodium. On essore l'insoluble, le lave avec une solution saturée au demi de chlorure de sodium puis à l'eau. On sèche et obtient 0,718 g de poudre blanche.

F = 132°C.
$[\alpha]_D^{20}$ = −83,5° ± 2° (c = 1% dans $CHCl_3$).

Analyse:

| | | | |
|---|---|---|---|
| Calculé: | C% 58,83 | H% 9,15 | N% 3,35 |
| Trouvé: | C% 59,0 | H% 8,9 | N% 3,2 |

## Exemple 18

### 9-[O-[(4-chloro phénylthio) méthyl] oxime] de l'érythromycine

On introduit sous argon 80 $cm^3$ d'éther éthylique et 2,99 g d'oxime de l'érythromycine. Après dissolution, sous agitation, on introduit 0,212 g d'hydrure de sodium à 50% dans l'huile puis 8 $cm^3$ d'hexaméthylphosphotriamide et 0,6 $cm^3$ de chlorure de 4-chloro phénylthio méthyle. Après 65 heures, on rajoute 0,15 $cm^3$ de chlorure et 4 heures plus tard, on verse le mélange dans une solution de 100 g d'acétate d'amyle dans 60 $cm^3$ d'eau. On extrait à l'éther, lave à l'eau les extraits éthérés, sèche et évapore à sec sous pression réduite. On obtient 3 g de résine. Les 3 g sont chromatographiés sur silice (éluant: toluène-triéthylamine 9—1). Les fractions sont réunies, séchées à température ambiante. On obtient 0,89 g d'un solide amorphe. 0,852 g de ce produit sont agités avec 13 $cm^3$ de pentane pendant 2 heures. On essore et lave 3 fois au pentane. Après séchage, on obtient 0,753 g de poudre blanche.

F = 126°C.
$[\alpha]_D$ = −32,5 ± 1,5° (c = 0,8% dans $CHCl_3$).

Analyse:

| | | | | | |
|---|---|---|---|---|---|
| Calculé: | C% 58,36 | H% 8,13 | N% 3,093 | S% 3,54 | Cl% 3,91 |
| Trouvé: | C% 58,6 | H% 8,1 | N% 3,0 | S% 3,7 | Cl% 4,2 |

## Exemple 19

### 9-[O-[2-(1,1-diméthyléthoxy) 2-oxo éthyl] oxime] de l'érythromycine

On dissout 3 g d'oxime d'érythromycine dans 80 $cm^3$ d'éther sec. On plonge dans un bain à 10°C et ajoute par petites fractions sous agitation 0,216 g d'hydrure de sodium à 50% dans l'huile. On introduit goutte à goutte après 8 minutes, 0,6 $cm^3$ de mono chloroacétate de tert-butyle et revient à température ambiante. On ajoute 8 $cm^3$ de diméthylformamide anhydre. Après 2 heures, on ajoute 0,044 g d'hydrure de sodium et 0,12 $cm^3$ de mono chloroacétate de ter-butyle. Après 1 heure, on fait barboter du gaz carbonique jusqu'à neutralité. On essore le mélange, le concentre et verse le résidu, sous agitation dans 160 $cm^3$ d'une solution aqueuse saturée en chlorure de sodium. One agite 1 heure, essore le précipité, le lave avec une solution saturée de chlorure de sodium, puis le sèche sous vide. On obtient 3,85 g de solide blanc. 3,7 g de ce produit sont chromatographiés sur silice avec élution par un mélange toluène-triéthylamine (9—1). On évapore à sec la partie homogène, triture le résidu amorphe obtenu avec du pentane et chasse le solvant sous pression réduite. On obtient 1,73 g d'une poudre blanche. F = 214°C. Les 1,73 g sont empâtés avec 5,1 $cm^3$ de pentane, essorés et lavés 2 fois au pentane. Après séchage sous vide, on obtient 1,599 g d'une poudre blanche.

F = 214°C.
$[\alpha]_D^{20}$ = −71,5° ± 2° à 1% dans le chloroforme.

Analyse:

| | | | |
|---|---|---|---|
| Calculé: | C% 59,84 | H% 9,11 | N% 3,23 |
| Trouvé: | C% 60,1 | H% 9,4 | N% 3,1 |

Exemple 20

9-[O-(éthoxyméthyl) oxime] de l'érythromycine

On introduit 3 g d'oxime d'érythromycine dans 10 $cm^3$ d'hexaméthylphosphotriamide. On rince par 2 $cm^3$ d'hexaméthylphosphotriamide. Après dissolution, on ajoute en 3 fractions 0,22 g d'hydrure de sodium à 50% dans l'huile. On ajoute en suite sous vive agitation 0,41 $cm^3$ de chlorométhyléthyl éther. On laisse agir 2 heures en tout. On verse dans 250 $cm^3$ d'une solution saturée aux $^3/_4$ de chlorure de sodium et contenant 2 g de bicarbonate de sodium. On rince avec 20 $cm^3$ de solution saturée aux $^3/_4$ de chlorure de sodium. On agite 30 minutes et laisse au repos. On essore le précipité obtenu, le lave avec la même solution, le met en solution dans le chloroforme, décante puis élimine l'insoluble par essorage. Le filtrat est lavé, séché, traité avec 0,8 g de charbon actif, élimine le solvant sous pression réduite et obtient 3,01 g d'une résine jaune. Ce produit est chromatographié sur silice (éluant: toluène-chloroforme-triéthylamine 6—4—1). On réunit les phases homogènes et évapore sous pression réduite. On obtient 2 g de résine blanche que l'on concrétise par trituration dans 3 $cm^3$ d'éther isopropylique pendant 20 minutes. On essore le produit, le rince, sèche et obtient 1,54 g d'un produit blanc.

F = 123°C.
$[\alpha]_D^{20}$ = −78° ± 2,5° (c = 1% dans le chloroforme).

Analyse:

| | | | |
|---|---|---|---|
| Calculé: | C% 59,53 | H% 9,01 | N% 3,48 |
| Trouvé: | C% 59,8 | H% 9,3 | N% 3,3 |

Exemple 21

9-[O-[(2-chloroéthoxy) méthyl] oxime] de l'érythromycine

On dissout sous argon 3 g d'oxime d'érythromycine dans 80 $cm^3$ d'éther sec. On plonge le ballon dans un bain à 10°C et introduit en 5 minutes par portions 0,216 g d'hydrure de sodium à 50% dans l'huile, puis 0,44 $cm^3$ de 1-chloroéthyl chlorométhyl éther. On laisse 5 heures 30 à température ambiante puis fait barboter du gaz carbonique. On évapore à sec sous pression réduite, reprend à l'éther de pétrole (Eb: 65—75°C), agite une demi-heure, puis essore et lave à l'éther de pétrole. Après séchage, on obtient 3,54 g de produit brut. 3,47 g de ce produit sont repris par 17,5 $cm^3$ d'acétone. On essore la suspension, agite le filtrat pendant une demi-heure avec 150 mg de charbon actif, puis essore. Le filtrat est de nouveau traité au charbon actif et concentré à sec. On obtient 3,21 g de produit. 2,93 g de ce produit sont agités une demi-heure avec 15 $cm^3$ d'eau à 10% d'éthanol. Le mélange est essoré et lavé par le même solvant (1,5; 1 et 1 $cm^3$). Après séchage, on obtient 2,67 g de produit.

F = 129°C environ.
$[\alpha]_D^{20}$ = −78,5° ± 3° (c = 0,5% dans le chloroforme).

Analyse:

| | | | | |
|---|---|---|---|---|
| Calculé: | C% 57,04 | H% 8,74 | Cl% 4,21 | N% 3,33 |
| Trouvé: | C% 56,8 | H% 8,7 | Cl% 4,3 | N% 3,2 |

Exemple 22

9-[O-[(2-diméthylaminoéthoxy) méthyl] oxime] de l'érythromycine

On mélange 8,86 g de diméthylamine avec 3 g de 9-[O-[(2-chloroéthoxy) méthyl] oxime] de l'érythromycine obtenue à l'exemple 21 refroidie dans un bain de glace. On ferme hermétiquement, plonge dans un bain à 30—32°C. Après dissolution complète, on laisse 74 heures à cette température. Après refroidissement au bain de glace, le récipient est ouvert. On élimine la diméthylamine. On sèche le résidu sous vide et obtient 3,286 g d'un solide blanc. 3,256 g de ce produit sont repris par 100 $cm^3$ d'acétate d'éthyle additionnés d'un peu d'éther et lavés à l'eau 6 fois, puis 3 fois avec une solution saturée de chlorure de sodium. La solution organique est séchée, évaporée puis le résidu est séché sous vide. On obtient 3,1 g de produit. 3 g de ce produit sont agités pendant 2 heures avec 10 $cm^3$ de

pentane. Par essorage et lavage puis séchage on obtient 2,79 g de produit.

F = 162°C.
$[\alpha]_D^{20}$ = −78° ± 2° à 1% dans le chloroforme.

Analyse:

| | | | |
|---|---|---|---|
| Calculé: | C% 59,34 | H% 9,37 | N% 4,94 |
| Trouvé: | C% 59,3 | H% 9,5 | N% 4,6 |

Exemple 23

9-[O-[(2-diéthylamino éthoxy) méthyl] oxime] de l'érythromycine

On place 2,5 g de 9-[O-[(2-chloréthoxy) méthyl] oxime] de l'érythromycine obtenue à l'exemple 21 et 10 $cm^3$ de l'érythromycine obtenue à l'exemple 21 et 10 $cm^3$ de diéthylamine dans un flacon que l'on ferme hermétiquement. On porte à 70°C pendant 48 heures puis ramène à température ambiante. On évapore à sec sous pression réduite. On obtient 3,1 g de résine que l'on chromatographie sur silice (éluant: toluène-chloroforme-triéthylamine 6—4—1). On réunit les fractions homogènes, évapore à sec sous pression réduite et obtient 1,5 g de résine blanche que l'on cristallise dans 5 $cm^3$ d'éther isopropylique. On obtient un premier jet de 0,924 g d'une poudre blanche. F = 135°C. Les liqueurs mères concentrées et abandonnées fournissent un deuxième jet de 0,22 g.

F = 122°C.
$[\alpha]_D^{20}$ = −74° ± 1,5° (c = 1% dans le chloroforme).

Analyse:

| | | | |
|---|---|---|---|
| Calculé: | C% 60,18 | H% 9,53 | N% 4,78 |
| Trouvé: | C% 60,4 | H% 9,6 | N% 4,7 |

Exemple 24

9-[O-[(2-aminoéthoxy) méthyl] oxime] de l'érythromycine

On refroidit 2,5 g de 9-[O-[(2-chloroéthoxy) méthyl] oxime] de l'érythromycine, obtenue à l'exemple 21, avec un mélange acétone-carboglace et, sous argon, on condense de l'ammoniac jusqu'aux 2/3 du récipient. On ferme hermétiquement, porte à 50°C pendant 20 heures puis refroidit dans un bain d'acétonecarboglace avant de réouvrir et de laisser l'ammoniac s'échapper. On reprend le résidu au chloroforme, évapore le solvant sous pression réduite et obtient 2,3 g de résine blanche que l'on chromatographie sur silice en éluant avec un mélange chloroforme-triéthylamine (9—1). On réunit les fractions homogènes. On évapore à sec, obtient 1,3 g de résine que l'on agite 20 minutes dans 3 $cm^3$ de pentane. On concrète, essore l'insoluble, le rince par 0,3 $cm^3$ de pentane, sèche à poids constant et obtient 0,92 g d'une poudre blanche.

F = 195°C.
$[\alpha]_D^{20}$ = −73° ± 2° (c = 1% dans le chloroforme).

Analyse:

| | | | |
|---|---|---|---|
| Calculé: | C% 58,44 | H% 9,19 | N% 5,11 |
| Trouvé: | C% 58,1 | H% 9,3 | N% 4,9 |

Exemple 25

9-[O-[[(2-(méthylamino) éthoxy] méthyl] oxime] de l'érythromycine

On refroidit à −70°C 2,5 g de 9-[O-[(2-chloroéthoxy) méthyl] oxime] de l'érythromycine obtenue à l'exemple 21 et condense 13,5 g de méthylamine dans un récipient hermétique et laisse remonter la température jusqu'à la température ambiante. On laisse la réaction se poursuivre pendant 41 heures. On refroidit de nouveau à −70°C, ouvre le récipient et laisse revenir à température ambiante sous agitation. La méthylamine s'évapore. On sèche sous pression réduite et obtient 2,7 g de résine blanche que l'on chromatographie sur silice (éluant: chloroforme-triéthylamine 92,5—7,5). On réunit les fractions homogènes et les évapore sous pression réduite. On obtient 2,2 g de résine blanche que l'on cristallise

dans 3 $cm^3$ d'éthanol. On essore le précipité, le rince par 0,3 $cm^3$ d'éthanol, sèche et obtient 1,7 g de poudre blanche.

F = 132°C.
$[\alpha]_D^{20}$ = −79° ± 2° (c = 1% dans le chloroforme).

Analyse:

| | |
|---|---|
| Calculé: | C% 58,9 H% 9,28 N% 5,03 |
| Trouvé: | C% 59,1 H% 9,4 N% 5,0 |

Exemple 26

9-[O-[[2-(morpholin-4-yl) éthoxy] méthyl] oxime] de l'érythromycine

On mélange sous agitation 2,5 g de 9-[O-[(2-chloréthoxy) méthyl] oxime] de l'érythromycine obtenue à l'exemple 21 et 10 $cm^3$ de morpholine dans un récipient hermétique et agite la solution pendant 6 jours à température ambiante. On évapore à sec et obtient 3 g de résine blanche que l'on chromatographie sur silice (éluant: toluène-chloroforme-triéthylamine 6—4—1). On réunit les fractions homogènes et les évapore à sec sous pression réduite. On obtient 2,4 g de résine blanche que l'on concrét par trituration dans 3 $cm^3$ d'un mélange pentaneéther isopropylique (1—1) pendant 20 minutes. On essore le produit obtenu, le rince par 0,3 $cm^3$ du même mélange pentaneéther isopropylique et sèche à poids constant. On obtient 1,72 g d'une poudre blanche.

F = 130°C.
$[\alpha]_D^{20}$ = −72° ± 1,5° (c = 1% dans le chloroforme).

Analyse:

| | |
|---|---|
| Calculé: | C% 59,24 H% 9,15 N% 4,71 |
| Trouvé: | C% 58,9 H% 9,1 N% 4,5 |

Exemple 27

9-[O-[(méthylthio) méthyl] oxime] de l'érythromycine

On introduit sous argon 3 g d'oxime d'érythromycine dans 80 $cm^3$ d'éther éthylique. Après dissolution, on introduit 0,212 g d'hydrude de sodium à 50% dans l'huile et agite 15 minutes supplémentaires. On ajoute alors en une seule fois 0,386 g de chlorométhyl méthyl sulfure dans 1 $cm^3$ d'éther. Au bout de 30 minutes, on rajoute 8 $cm^3$ de diméthylformamide. Au bout d'1 heure et demie, on rajoute de nouveau 8 $cm^3$ de diméthylformamide. Après une nuit, on rajoute 70 mg d'hydrure de sodium et 0,129 g de chlorométhyl méthyl sulfure. Une heure et demie après, on sature sous violent barbotage de gaz carbonique pendant 1 heure et demie et précipite par 300 $cm^3$ d'une solution saturée de chlorure de sodium. On essore l'insoluble, le regrend par du chloroforme, lave avec une solution de chlorure de sodium, sèche et évapore à sec. On obtient 4,045 g de produit brut que l'on chromatographie sur silice (éluant: toluène-triéthylamine 9—1). On rassemble les fractions homogènes les évapore à sec sous vide et obtient 1,3 g d'une résine blanche qui cristallise par trituration dans 7 $cm^3$ d'éther de pétrole (Eb: 65—75°C). On essore le produit formé, le rince par 0,7 $cm^3$ d'éther de pétrole et sèche. On obtient 0,93 g de poudre blanche.

F = 132°C
$[\alpha]_D^{20}$ = −75,5° ± 2,5° (c = 0,45% dans le chloroforme).

Analyse

| | |
|---|---|
| Calculé: | C% 57,9 H% 8,97 N% 3,46 S% 3,96 |
| Trouvé: | C% 57,6 H% 8,9 N% 3,4 S% 4,0 |

Exemple 28

9-[O-[(phénylthio) méthyl] oxime] de l'érythromycine

On dissout 3 g d'oxime d'érythromycine dans 10 $cm^3$ d'éther et 10 $cm^3$ d'hexaméthylphosphotriamide. On évapore l'éther sous pression réduite. On place sous argon et ajoute en plusieurs fois, à température ambiante 0,212 g d'hydrure de sodium à 50%. On agite 15 minutes et ajoute goutte à

goutte 0,6 $cm^3$ de chlorométhyl phényl sulfure. On arrête la réaction 1 heure 10 minutes après la fin de l'introduction en versant le mélange dans 240 $cm^3$ d'une solution de chlorure de sodium saturée aux $^3/_4$ et contenant 1 g de carbonate acide de sodium. On agite 15 minutes à température ambiante, essore le précipité formé et le rince 1 fois à l'eau. On le dissout dans du chloroforme, lave avec une solution saturée de chlorure de sodium, sèche et évapore à sec sous pression réduite. On obtient 4,48 g d'une résine jaune pâle que l'on chromatographie sur silice (éluant: chloroforme-triéthylamine 9—1). On réunit les fractions homogènes et les évapore à sec. On obtient 3,2 g d'une résine blanche que l'on cristallise dans 10 $cm^3$ de pentane. On agite 30 minutes à température ambiante, essore le produit formé et le rince par 0,5 $cm^3$ de pentane. On sèche et obtient 2,39 g d'une poudre blanche.

F = 198°C
$[\alpha]_D^{20}$ = −2,5° ± 1° (c = 0,5% dans le chloroforme).

Analyse

| | | | | |
|---|---|---|---|---|
| Trouvé: | C% 60,6 | H% 8,6 | N% 3,3 | S% 3,7 |
| Calculé: | C% 60,66 | H% 8,56 | N% 3,21 | S% 3,68 |

## Exemple 29

### 9-[O-(2,2-diméthoxyéthyl) oxime] de l'érythromycine

On dissout 3 g d'oxime d'érythromycine dans un mélange de 10 $cm^3$ d'éther et 9,6 $cm^3$ d'hexaméthylphosphotriamide. On chasse l'éther sous vide et ramène à température ambiante. On ajoute alors en plusieurs fois 0,212 g d'hydrure de sodium à 50% dans l'huile, agite 15 minutes et ajoute goutte à goutte 0,6 $cm^3$ de bromoacétaldéhyde diméthyl acétal. On arrête la réaction au bout de 2 heures en la versant dans 240 $cm^3$ d'une solution saturée aux $^3/_4$ de chlorure de sodium et contenant 1 g de carbonate acide de sodium. On agite 10 minutes, essore le précipité, le rince avec la solution saturée aux $^3/_4$ de chlorure de sodium, le lave avec une quantité suffisante de chloroforme pour obtenir la solubilisation de la partie organique. On sèche le filtrat, évapore à sec sous pression réduite et obtient 5 g de résine blanche que l'on chromatographie sur silice (éluant: toluène-triéthylamine 9—1). On obtient 3 g de résine que l'on cristallise dans 5 $cm^3$ de pentane. On essore le produit formé, le rince par 0,5 $cm^3$ de pentane, sèche sous pression réduite et obtient 1,912 g de produit attendu.

$F_1$ = 146°C.
$F_2$ = 186°C.
$[\alpha]_D^{20}$ = −80° ± 2,5° (c = 0,6% dans le chloroforme).

Analyse

| | | | |
|---|---|---|---|
| Calculé: | C% 58,83 | H% 9,15 | N% 3,35 |
| Trouvé: | C% 59,0 | H% 9,3 | N% 3,3 |

## Exemple 30

### 9-[O-[(1,3-dioxolan-2-yl) méthyl] oxime] de l'érythromycine

On introduit 3 g d'oxime d'érythromycine dans 60 $cm^3$ d'éther. On ajoute ensuite en plusieurs fois 0,212 g d'hydrure de sodium à 50% dans l'huile, puis 6 $cm^3$ de diméthylformamide. On poursuit l'agitation 15 minutes puis ajoute goutte à goutte 0,6 $cm^3$ de 2-bromométhyl 1,3-dioxolanne. Au bout de 3 heures, on évapore l'éther sous argon et rince par 1 $cm^3$ de diméthylformamide. On laisse une nuit à température ambiante sous agitation. Après 19 heures de réaction, on rajoute 0,106 g d'hydrure sodium et 0,15 $cm^3$ de 2-bromométhyl 1,3-dioxolanne. 5 heures après, on arrête la réaction par barbotage de gaz carbonique pendant 1 heure puis verse dans 140 $cm^3$ d'une solution saturée de chlorure de sodium. On agite 10 minutes, essore le précipité formé et le solubilise dans le chloroforme. On sèche la solution, évapore à sec sous pression réduite et obtient 3,7 g de résine que l'on chromatographie sur silice (éluant: toluène-triéthylamine 0—1). On réunit les fractions homogènes et les évapore à sec. On obtient 1,55 g de résine blanche que l'on concrète dans 5 $cm^3$ de pentane. On essore le précipité formé et le rince par 0,5 $cm^3$ de pentane. Après séchage, on obtient 1,227 g de produit.

$F_1$ = 140°C.
$F_2$ = 208°C.
$[\alpha]_D^{20}$ = −67,5° ± 1,5° (c = 1% dans le chloroforme).

Analyse
Calculé: C% 58,91 H% 8,93 N% 3,35
Trouvé: C% 59 H% 8,9 N% 3,3

## Exemple 31

9-[O-[2-(diéthylamino) éthyl] oxime] de l'érythromycine

On dissout 5,62 g d'oxime d'érythromycine dans 50 $cm^3$ de tétrahydrofuranne, ajoute 1 $cm^3$ d'une solution 0,04 M de carbonate de sodium puis 2,58 g de chlorhydrate de 2-chloro 1-diéthylamino-éthane. On porte au reflux le mélange réactionnel pendant 16 heures. On élimine l'insoluble et le filtrat est traité au charbon actif puis concentré à sec pour donner 6,21 g de produit brut. Après recristallisation dans un mélange isopropanol-eau, on obtient 5,82 g de cristaux incolores.

F ≃ 220°C.
$[\alpha]_D^{20}$ = −83° ± 2,5° (c = 0,7% EtOH).

Analyse: $C_{43}H_{81}N_3O_{13}$
Calculé: C% 60,9 H% 9,6 N% 4,95
Trouvé: C% 61,0 H% 9,8 N% 4,8

## Exemple 32

9-[O-[2-(pyrrolidin-1-yl) éthyl] oxime] de l'érythromycine

On dissout 2,25 g d'oxime d'érythromycine dans 50 $cm^3$ d'acétone anhydre, ajoute 0,720 g de carbonate de sodium et 0,76 g de chlorhydrate de N-(β-chloroéthyl) pyrrolidine). On porte au reflux pendant 6 jours. On ajoute 40 $cm^3$ d'acétone et filtre l'insoluble à chaud. On verse 100 $cm^3$ d'eau distillée dans le filtrat et obtient par essorage 2 g de produit brut que l'on purifie par passage sur silice avec un mélange éther-triéthylamine (9—1). On recueille 1,72 g de produit attendu.

$[\alpha]_D$ = −83° ± 2° (c = 1% EtOH).

## Exemple 33

9-[O-[méthoxyméthyl] oxime] de l'érythromycine

On dissout 3,75 g d'oxime d'érythromycine dans 75 $cm^3$ d'acétone, ajoute 2,1 g de bicarbonate de sodium et 1,3 g de chlorométhyl méthyl éther. On porte le mélange au reflux pendant 4 jours, en ajoutant à 17 heures 30 et à 48 heures, 2 fois 0,45 $cm^3$ de réactif. On verse le mélange réactionnel dans 100 $cm^3$ d'eau et extrait au chloroforme. On lave la phase organique avec une solution aqueuse saturée de chlorure de sodium, sèche, concentre à sec sous pression réduite. On chromatographie le résidu sur silice, élue par un mélange benzène- chloroforme-triéthylamine (5—4—1) et obtient 2,06 g de produit attendu.

$[\alpha]_D$ = −80,5° ± 2,5° (c = 0,6% éthanol).

Le chlorométhyl méthyl éther a été préparé comme suit:
On agite 45 $cm^3$ de diméthoxyméthane avec 1,2 $cm^3$ de méthanol anhydre et ajoute goutte à goutte 35,5 $cm^3$ de chlorure d'acétyle en maintenant la température aux environs de 20°C. On agite ainsi pendant 3 jours puis utilise le réactif obtenu, tel quel.

## Exemple 34

9-[O-[2-(pipéridin-1-yl) éthyl] oxime] de l'érythromycine

On dissout 2,25 g d'oxime d'érythromycine dans 50 $cm^3$ d'acétone anhydre, ajoute 0,72 g de carbonate de sodium et 0,830 g de chlorhydrate de 2-chloroéthyl N-pipéridine. On porte le mélange au reflux pendant 4 jours. Ensuite on ajoute 40 $cm^3$ d'acétone, porte à nouveau au reflux, filtre à chaud

l'insoluble puis évapore à sec le filtrat. On chromatographie le résidu sur silice, élue par un mélange benzène-chloroformetriéthylamine (5—4—1) et obtient 1,7 g de produit attendu.

$[\alpha]_D$ = −78° ± 2,5° (c = 0,6% éthanol).

Exemple 35

9-[O-[2-(morpholin-4-yl) éthyl] oxime] de l'érythromycine

On dissout 2,25 g d'oxime d'érythromycine dans 60 $cm^3$ d'acétone, ajoute 0,93 g de carbonate de sodium et 1,120 g de chlorhydrate de N-$\beta$-chloroéthyl morpholine. On porte le mélange réactionnel au reflux pendant 6 jours. On ajoute 30 $cm^3$ d'acétone, porte de nouveau au reflux, filtre à chaud l'insoluble et concentre à sec le filtrat sous pression réduite. On reprend par de l'acétone à froid, filtre le produit brut. Après dissolution dans l'acétone, concentration à faible volume puis addition d'éther, on essore, sèche et obtient 1,45 g de produit attendu.

$[\alpha]_D$ = −73,5° ± 3° (c = 0,5% éthanol).

Analyse: $C_{43}H_{79}N_3O_{14}$ = 862,112

| | | | |
|---|---|---|---|
| Calculé: | C% 59,9 | H% 9,2 | N% 4,87 |
| Trouvé: | C% 59,9 | H% 9,3 | N% 4,7 |

Exemple 36

9-[O-[2-(diméthylthio) 1-méthyl éthyl] oxime] de l'érythromycine

On dissout 20 g d'oxime d'érythromycine dans 300 $cm^3$ d'acétone, ajoute 6,4 g de carbonate de sodium et 6,35 g de 2-chloro 1-diméthylamino propane. On porte le mélange réactionnel au reflux pendant 3 jours. On ajoute 100 $cm^3$ d'acétone et filtre l'insoluble. On traite le filtrat au charbon actif et ajoute 500 $cm^3$ d'eau. On essore le produit cristallisé et obtient 19,8 g de produit attendu.

$[\alpha]_D$ = −96° (c = 0,75% éthanol).

Analyse: $C_{42}H_{79}N_3O_{13}$ = 834,10

| | | | |
|---|---|---|---|
| Calculé: | C% 60,48 | H% 9,54 | N% 5,03 |
| Trouvé: | C% 60,3 | H% 9,6 | N% 5,0 |

Exemple 37

9-[O-[(2-diméthylamino) propyl] oxime] de l'érythromycine

On dissout 3,75 g d'oxime d'érythromycine dans 40 $cm^3$ d'acétone, ajoute 1,2 g de carbonate de sodium et 1,22 g de chlorhydrate de 3-chloro diméthylamino propyle. On porte le mélange réactionnel au reflux pendant 7 jours. On ajoute 50 $cm^3$ d'acétone, chauffe à nouveau et filtre à chaud l'insoluble. On concentre le filtrat jusqu'à 5 $cm^3$ environ, ajoute 100 $cm^3$ d'éther de pétrole (Eb: 60—80°C) et essore le produit brut. On le dissout dans 60 $cm^3$ environ d'acétone, traite au charbon actif à 40°C, filtre. On concentre le filtrat à petit volume, ajoute peu à peu de l'éther de pétrole (Eb: 60—80°C) puis laisse refroidir à température ambiante. On essore 1,30 g de produit attendu.

$[\alpha]_D$ = −70° ± 2° (c = 1% $CHCl_3$).

Analyse: $C_{42}H_{79}N_3O_{13}$ = 834,109

| | | | |
|---|---|---|---|
| Calculé: | C% 60,5 | H% 9,5 | N% 5,0 |
| Trouvé: | C% 60,6 | H% 9,7 | N% 4,9 |

Exemple 38

9-[O-(2-bromoéthyl) oxime] de l'érythromycine

On mélange 5,62 g d'oxime d'érythromycine et 100 $cm^3$ d'éther. On ajoute 0,75 g d'hydrure de sodium et, après la fin du dégagement gazeux, 15 $cm^3$ de 1,2-dibromoéthane. On porte le mélange

réactionnel au reflux pendant 16 heures, refroidit à 20°C pour ajouter 0,75 g d'hydrure de sodium, puis chauffe à nouveau au reflux pendant 6 heures. On refroidit le mélange à 0°C environ, ajoute 100 $cm^3$ de chlorure de méthylène et goutte à goutte, en agitant, 3 $cm^3$ d'acide acétique. On laisse alors revenir à température ambiante et verse le mélange réactionnel dans 50 $cm^3$ d'eau contenant 4 $cm^3$ d'ammoniaque à 28%. On sépare la phase organique, extrait la phase aqueuse au chlorure de méthylène. Les phases organiques sont réunies, lavées à l'eau, séchées et concentrées à sec. On chromatographie le résidu obtenu sur silice, élue par le mélange chlorure de méthylène-triéthylamine (25—0,5) et obtient 4,78 g de produit attendu.

$[\alpha]_D$ = −87° ± 2,5° (c = 0,5% EtOH).

Analyse: $C_{39}H_{71}BrN_2O_{13}$ = 855,9

| | | | | |
|---|---|---|---|---|
| Calculé: | C% 54,7 | H% 8,3 | N% 3,27 | Br 9,3 |
| Trouvé: | C% 54,6 | H% 8,3 | N% 3,2 | Br 9,2 |

Exemple 39

9-[O-[2-[(1-méthyléthyl) amino] éthyl] oxime] de l'érythromycine

Pendant 15 heures, on agite 2,58 g de 9-[O-(2-bromoéthyl) oxime] de l'érythromycine dans 12 $cm^3$ d'isopropylamine à température ambiante et sous atmosphère inerte. On verse le mélange réactionnel dans 600 $cm^3$ d'eau, essore le précipité obtenu, le lave à l'eau, le sèche et obtient 2,29 g de produit brut. On le purifie par chromatographie sur silice en éluant par un mélange chlorure de méthylène-triéthylamine (25—0,5). On recueille 1,60 g de produit attendu.

$[\alpha]_D$ = −78,5° (c = 0,5% EtOH).

Analyse: $C_{42}H_{79}N_3O_{13}$ = 834

| | | | |
|---|---|---|---|
| Calculé: | C% 60,5 | H% 9,55 | N% 5,04 |
| Trouvé: | C% 60,6 | H% 9,5 | N% 4,9 |

Exemple 40

9-[O-[(2-méthyl propoxy) méthyl] oxime] de l'érythromycine

On dissout 4,49 g d'oxime d'érythromycine dans 40 $cm^3$ d'acétone, ajoute 2,52 g de bicarbonate de sodium et 0,76 g de chlorométhyl isobutyl éther. On porte le mélange réactionnel au reflux pendant 41 heures, en ajoutant à 18 heures et à 26 heures 2 fois 0,4 $cm^3$ de chlorométhyl isobutyl éther. On filtre l'insoluble et concentre à sec le filtrat. On reprend le résidu par 20 $cm^3$ d'une solution aqueuse saturée de bicarbonate de sodium, essore l'insoluble, le purifie par chromatographie sur silice en éluant par un mélange benzène-triéthylamine (15—1). On obtient 3,07 g de produit attendu.

$[\alpha]_D$ = −79,5° ± 3° (c = 0,5% EtOH).

Analyse: $C_{42}H_{78}N_2O_{14}$ = 835

| | | | |
|---|---|---|---|
| Calculé: | C% 60,4 | H% 9,4 | N% 3,35 |
| Trouvé: | C% 60,7 | H% 9,7 | N% 3,5 |

Exemple 41

9-[O-[2-[bis (1-méthyléthyl) amino] éthyl] oxime] de l'érythromycine

On dissout 5,62 g d'oxime d'érythromycine dans 50 $cm^3$ de tétrahydrofuranne, ajoute 5,04 g de bicarbonate de sodium et 1,25 g de chlorhydrate de 2-chloro 1-[bis (1-méthyléthyl) amino] éthane. On chauffe au reflux le mélange réactionnel pendant 4 jours en ajoutant après 64 heures 0,82 g de chlorhydrate de 2-chloro 1-[bis (1-méthyléthyl) amino] éthane. On filtre l'insoluble et concentre à sec le filtrat. Le résidu obtenu est chromatographié sur silice. On élue par un mélange chlorure de méthylène-triéthylamine (25—0,5) et obtient 4,37 g de produit attendu.

$[\alpha]_D$ = −73° ± 3° (c = 0,5% EtOH).

Analyse: $C_{45}H_{85}N_3O_{13}$ = 876
Calculé: C% 61,7 H% 9,8 N% 4,8
Trouvé: C% 61,7 H% 9,9 N% 4,7

## Exemple 42

### 9-[O-[2-(diéthoxy) éthyl] oxime] de l'érythromycine

On dissout 5,62 g d'oxime de l'érythromycine dans un mélange de 18 $cm^3$ d'hexaméthylphosphotriamide et 5 $cm^3$ d'éther éthylique. On ajoute 0,4 g d'hydrure de sodium à 50% dans l'huile, agite pendant 15 minutes puis ajoute goutte à goutte 1,55 $cm^3$ de bromoacétaldéhyde diéthylacétal. On agite pendant 3 heures 30 minutes à 20°C, puis verse dans 500 $cm^3$ d'une solution aqueuse de chlorure de sodium renfermant 2 g de bicarbonate de sodium. On extrait à l'éther, lave la phase éthérée à l'eau salée, la sèche et la concentre à sec. On obtient 6,35 g de produit brut que l'on chromatographie sur silice en éluant au mélange benzène-triéthylamine (9—1), puis au mélange benzène-chloroforme-triéthylamine (7—2—1) et obtient 1,7 g de produit attendu.

$[\alpha]_D$ = −75,5° ± 2,5° (c = 0,65% éthanol).

Analyse: $C_{43}H_{80}N_2O_{15}$ = 865,120
Calculé: C% 59,70 H% 9,32 N% 3,23
Trouvé: C% 60,1 H% 9,4 N% 3,3

## Exemple 43

### 9-[O-[2-(4-méthylpipérazin-1-yl) éthyl] oxime] de l'érythromycine

On dissout 2,31 g du dérivé bromé, obtenu à l'exemple 38, dans 10 $cm^3$ de N-méthylpipérazine et agite à 20°C pendant 20 heures. On verse dans 50 $cm^3$ d'eau renfermant 0,5 g de carbonate de potassium, filtre le précipité, le lave à l'eau, le sèche et obtient 1,99 g de produit brut. On dissout le produit dans le chlorure de méthylène, lave avec une solution aqueuse de carbonate de sodium, sèche, concentre à sec, reprend à l'éther isopropylique, filtre, élimine le solvant et lave le résidu au pentane. On obtient 0,94 g de produit attendu.

$[\alpha]_D$ = −81° ± 2° (c = 1% éthanol).

Analyse: $C_{44}H_{82}N_4O_{13}$ = 875,2
Calculé: C% 60,4 H% 9,45 N% 6,40
Trouvé: C% 60,5 H% 9,4 N% 6,2

## Exemple 44

### 9-[O-[2-(éthylamino) éthyl] oxime] de l'érythromycine

On introduit 2,31 g du dérivé bromé obtenu à l'exemple 38, dans un récipient de 50 $cm^3$, refroidit à l'aide d'un mélange acétone-carboglace, puis introduit de la mono éthylamine jusqu'aux $^2/_3$ du volume. On ferme hermétiquement, laisse revenir à la température ambiante en agitant, poursuit l'agitation pendant 20 heures, refroidit, ouvre le récipient, élimine la monoéthylamine, reprend le résidu à l'eau, filtre, lave à l'eau, sèche et obtient 2,3 g de produit brut que l'on chromatographie sur silice en éluant au mélange chlorure de méthylène-triéthylamine (25—0,5) et obtient 1,76 g de produit attendu.

$[\alpha]_D$ = −82° ± 2° (c = 1% éthanol).

Analyse: $C_{41}H_{77}N_3O_{13}$ = 820
Calculé: C% 60,05 H% 9,47 N% 5,12
Trouvé: C% 60,5 H% 9,5 N% 4,7

## Exemple 45

### 9-[O-(2-amino éthyl) oxime] de l'érythromycine

On introduit 4,5 g du dérivé bromé obtenu à l'exemple 38, dans un récipient de 50 $cm^3$. On refroidit à l'aide d'un mélange acétone-carboglace et introduit 20 $cm^3$ d'ammoniac. On ferme hermétiquement, laisse revenir la température à 20°C et maintient sous agitation pendant 68 heures. On refroidit, élimine l'ammoniac, reprend au chlorure de méthylène, lave à l'eau, sèche et évapore à sec. On obtient 3,97 g de produit brut que l'on chromatographie sur silice en éluant au mélange chlorure de méthylène-triéthylamine (19—1). On reprend le produit obtenu au chlorure de méthylène, lave la solution par une solution aqueuse de carbonate de sodium, sèche et concentre à sec. On chromatographie à nouveau le résidu sur silice en éluant au mélange chloroforme-triéthylamine (15—1) et obtient 1,67 g de produit attendu.

$[\alpha]_D$ = −76° ± 2° (c = 0,8% éthanol).

Analyse: $C_{39}H_{73}N_3O_{13}$ = 792

| | | | |
|---|---|---|---|
| Calculé: | C% 59,1 | H% 9,3 | N% 5,3 |
| Trouvé: | C% 59,4 | H% 9,4 | N% 5,2 |

## Exemple 46

### 9-[O-[2-(diéthylamino) 1-méthyléthyl] oxime] de l'érythromycine

On dissout 2 g d'oxime d'érythromycine dans 30 $cm^3$ d'acétone. On ajoute 0,64 g de carbonate de sodium puis 0,8 g de chlorhydrate de 1-diéthylamino 2-chloropropane et porte au reflux pendant 23 heures. On filtre les sels minéraux, ajoute de l'eau au filtrat, essore les cristaux, les lave à l'aide d'une solution acétone-eau, les sèche et obtient 1,9 g de produit attendu.

$[\alpha]_D$ = −84° ± 2,5° (c = 0,75% éthanol).

Analyse: $C_{44}H_{83}N_3O_{13}$ = 862,164

| | | | |
|---|---|---|---|
| Calculé: | C% 61,3 | H% 9,7 | N% 4,87 |
| Trouvé: | C% 61,4 | H% 9,7 | N% 4,8 |

## Exemple 47

### 9-[O-(oxiramyl méthyl) oxime] de l'érythromycine

On dissout 5 g d'oxime d'érythromycine dans 23 $cm^3$ de diméthylformamide puis ajoute lentement 354 mg d'hydrure de sodium à 50% dans l'huile, agite pendant 15 minutes, puis ajoute goutte à goutte 1 g d'épibromohydrine dilué dans 1 $cm^3$ de diméthylformamide. On neutralise ensuite par barbotage de gaz carbonique, verse le mélange dans 250 $cm^3$ d'une solution aqueuse saturée de chlorure de sodium, agite pendant 10 minutes puis essore le précipité formé. On le rince à l'eau puis le dissout dans le chloroforme, sèche, traite au charbon actif, concentre à sec et cristallise dans le pentane. On obtient 4,1 g de produit brut que l'on chromatographie sur silice en éluant au mélange chloroforme-triéthylamine (9—1). On obtient après cristallisation dans le pentane, 0,87 g de produit attendu.

F = 154°C.
$[\alpha]_D$ = −79° ± 2,5° (c = 0,5% chloroforme).

Analyse: $C_{40}H_{72}N_2O_{14}$ = 805

| | | | |
|---|---|---|---|
| Calculé: | C% 59,68 | H% 9,01 | N% 3,48 |
| Trouvé: | C% 59,6 | H% 9,1 | N% 3,5 |

## Exemple 48

### 9-[O-[3-(diméthylamino) 2-hydroxypropyl] oxime] de l'érythromycine

On introduit 2,5 g de produit obtenu à l'exemple 47 dans un récipient que l'on ferme hermétiquement et refroidit dans un bain de glace. On introduit rapidement 2,8 g de diméthylamine, referme le récipient et agite à 20°C pendant 4 heures 10 minutes. On refroidit à nouveau, ouvre le récipient et élimine la diméthylamine. On chromatographie le résidu sur silice en éluant au mélange chloroforme-triéthylamine (9—1) et obtient après cristallisation dans le pentane et séchage, 1,21 g de produit attendu.

F = 144°C.
$[\alpha]_D^{20}$ = −84,5° ± 2,5° (c = 0,6% chloroforme).

Analyse: $C_{42}H_{79}N_3O_{14}$ = 850,10
Calculé: C% 59,34 H% 9,37 N% 4,94
Trouvé: C% 59,1 H% 9,4 N% 4,8

## Exemple 49

### 9-[O-[2-(diéthylamino) éthyl] oxime] de l'érythromycine (Autre isomère au niveau de l'oxime que le produit de l'exemple 31)

On utilise au départ 4,5 g d'oxime d'érythromycine (isomère différent de celui utilisé à l'exemple 31) que l'on dissout dans 45 $cm^3$ de tétrahydrofuranne. On ajoute 2,6 g de carbonate de sodium puis 2,1 g de chlorhydrate de chlorure de diéthylaminoéthyle. On porte au reflux pendant 5 heures 30 minutes, filtre, concentre le filtrat à sec et chromatographie le résidu sur silice en éluant au mélange benzène-chloroforme-triéthylamine (5—4—1), puis au mélange chlorure de méthylène-triéthylamine (15—1). On obtient 0,5 g de produit attendu.

$[\alpha]_D$ = −52° ± 2,5° (c = 0,5% éthanol).

Analyse: $C_{43}H_{81}N_3O_{13}$
Calculé: C% 60,89 H% 9,62 N% 4,95
Trouvé: C% 61,2 H% 9,7 N% 4,9

## Etude pharmacologique des produits de l'invention

### A) Activité in vitro

### Méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par transillumination ce qui permet de déterminer les concentrations minimales inhibitrices (C. M. I.) exprimées en µg/$cm^3$.

Les résultats suivants ont été obtenus:

C. M. I. à 24 heures

| Souches | Produits | | | | | |
|---|---|---|---|---|---|---|
| | Ex 1 et 6 | Ex 2 | Ex 3 | Ex 4 | Ex 5 | Ex 7 |
| Staphylococcus aureus ATCC 6538 Pen-Sensibles | 0,5 | 0,1 | 0,5 | 2 | 1 | 0,2 |
| Staphylococcus aureus UC 1128 Pen-Résistant | 2 | 1 | 1 | 3 | 1 | 1 |
| Staphylococcus aureus exp. n° 54 146 | 1 | 0,5 | 0,5 | 2 | 1 | 0,5 |
| Streptococcus pyogènes A 561 | 0,05 | ≤0,02 | 0,1 | 0,05 | ≤0,02 | 0,05 |
| Streptococcus faecalis 5432 | 0,2 | 0,1 | 0,2 | 0,5 | 0,05 | 0,1 |
| Streptococcus faecalis 99 F 74 | 0,2 | 0,1 | 0,2 | 0,2 | 0,05 | 0,1 |
| Bacillus subtilis ATCC 6633 | 0,2 | 0,2 | 0,2 | 0,5 | 0,5 | 0,1 |

C. M. I. à 24 heures

| Souches | Produits des exemples | | | | | | | | | | |
|---|---|---|---|---|---|---|---|---|---|---|---|
| | 13 | 14 | 15 | 16 | 17 | 20 | 21 | 22 | 23 | 24 | 25 |
| Staphylococcus aureus 1061 pen-sensibles | 0,4 | 0,2 | 0,6 | 0,4 | 0,4 | 0,4 | 0,2 | 0,4 | 0,4 | 0,1 | 0,5 |
| Staphylococcus aureus UC 1128 pen-résistants | 0,6 | 0,4 | 1 | 0,6 | 0,6 | 0,6 | 0,2 | 0,6 | 1 | 0,4 | 0,5 |
| Staphylococcus aureus exp. 54 146 | 0,4 | 0,4 | 0,6 | 0,4 | 0,4 | 0,4 | 0,2 | 0,4 | 0,6 | 0,4 | 0,5 |
| Streptococcus pyogenes A 561 | 0,02 | 0,02 | 0,1 | 0,05 | 0,05 | 0,1 | 0,02 | ≤0,01 | 0,02 | ≤0,01 | ≤0,01 |
| Streptococcus faecalis 5432 | 0,01 | 0,1 | 0,1 | 0,2 | 0,1 | 0,1 | 0,1 | 0,05 | 0,1 | 0,05 | 0,05 |
| Streptococcus faecalis 99 F 74 | 0,1 | 0,05 | 0,2 | 0,1 | 0,1 | 0,1 | 0,1 | 0,05 | 0,1 | 0,1 | 0,05 |
| Streptococcus faecalis 5435 | 0,4 | 0,4 | 0,6 | 1 | 0,6 | 0,2 | – | 0,2 | 0,2 | 0,2 | 0,2 |

C. M. I. à 24 heures

| Souches | Produits des exemples | | | | | | | | | |
|---|---|---|---|---|---|---|---|---|---|---|
| | 26 | 28 | 29 | 30 | 31 | 32 | 33 | 34 | 35 | 36 |
| Staphylococcus aureus 1061 pen-sensibles | 0,6 | 0,4 | 0,4 | 0,4 | 0,5 | 0,4 | 0,1 | 0,4 | 0,4 | 0,2 |
| Staphylococcus aureus UC 1128 pen-résistants | 0,1 | 0,6 | 1 | 0,6 | 1 | 1 | 0,4 | 0,6 | 1 | 0,5 |
| Staphylococcus aureus exp. 54 146 | 0,6 | 0,6 | 0,4 | 0,4 | 1 | 0,6 | 0,2 | 0,6 | 0,6 | 0,5 |
| Streptococcus pyogenes A 561 | 0,1 | 0,05 | 0,1 | 0,05 | 0,02 | ≤0,01 | 0,02 | ≤0,01 | 0,05 | ≤0,01 |
| Streptococcus faecalis 5432 | 0,2 | 0,2 | 0,4 | 0,4 | 0,1 | 0,05 | 0,1 | 0,1 | 0,2 | 0,1 |
| Streptococcus faecalis 99 F 74 | 0,2 | 0,2 | 0,2 | 0,2 | 0,1 | 0,1 | 0,1 | 0,05 | 0,4 | 0,1 |
| Streptococcus faecalis 5435 | 1 | 0,4 | 1 | 2 | – | 0,2 | 0,2 | 0,2 | 1 | 0,5 |

C. M. I. à 24 heures

| Souches | Produits des exemples | | | | | | |
|---|---|---|---|---|---|---|---|
| | 39 | 41 | 43 | 45 | 46 | 47 | 48 |
| Staphylococcus aureus 1061 pen-sensibles | 0,3 | 0,5 | 1 | 0,6 | 0,2 | 0,2 | 0,4 |
| Staphylococcus aureus UC 1128 pen-résistants | 0,6 | 1 | 2 | 1 | 0,6 | 0,4 | 0,6 |
| Staphylococcus aureus exp. 54 146 | 0,4 | 1 | 1 | 1 | 0,4 | 0,4 | 0,6 |
| Streptococcus pyogenes A 561 | 0,01 | 0,05 | ≤0,01 | ≤0,01 | ≤0,01 | 0,05 | 0,05 |
| Streptococcus faecalis 5432 | 0,05 | 0,02 | 0,2 | 0,4 | 0,05 | 0,2 | 0,4 |
| Streptococcus faecalis 99 F 74 | ≤0,02 | 0,2 | 0,05 | 0,1 | 0,05 | 0,2 | 0,1 |
| Streptococcus faecalis 5435 | 0,1 | – | 0,4 | 0,2 | 0,1 | 0,2 | 1 |

B) Activité in vivo

a) Infection expérimentale à Staphylococcus aureus exp. n° 54 146.

On a étudié l'action de quelques produits des exemples et de l'érythromycine sur une infection expérimentale à Staphylococcus aureus de la souris.

On a infesté des lots de dix souris mâles d'un poids moyen de 21 g par injection intrapéritonéale de 0,5 $cm^3$ d'une culture de 22 heures en bouillon à pH 7 de la souche de Staphylococcus aureus N° 54 146, diluée au 1/6 par de l'eau physiologique.

On a administré per os une heure, cinq heures et 24 heures après l'infection une quantité déterminée de produit.

On a noté la mortalité au 8ème jour.
Les résultats ont été les suivants:

| Produits | Doses<br>Souris survivantes — au 8ème jour | | | | | |
|---|---|---|---|---|---|---|
| | 0,25 mg | 0,5 mg | 0,75 mg | 1 mg | 1,5 mg | 2 mg |
| Produit ex 1 | 7 | 10 | | | | |
| Produit ex 3 | | 7 | 9 | 9 | | |
| Produit ex 4 | 0 | 4 | 9 | 10 | | |
| Produit ex 5 | 2 | 8 | 10 | | | |
| Produit ex 15 | 1 | 9 | 10 | | | |
| Produit ex 20 | 2 | 4 | 10 | | | |
| Produit ex 21 | 0 | 6 | 10 | | | |
| Produit ex 29 | 2 | 8 | 9 | 10 | | |
| Produit ex 30 | 2 | 7 | 10 | | | |
| Produit ex 31 | 4 | 10 | | | | |
| Produit ex 33 | 7 | 10 | | | | |
| Produit ex 35 | 2 | 8 | 9 | 10 | | |
| Produit ex 36 | 3 | 9 | 10 | | | |
| Produit ex 41 | 9 | 10 | | | | |
| Erythromycine | | | 5 | 9 | 9 | 10 |

b) Infection expérimentale à Streptococcus pyogènes A 561.

On a étudié l'action des produits des exemples 1 et 5 sur une infection expérimentale à Streptococcus pyogènes A 561 de la souris.

On a infesté des lots de 10 souris mâles d'un poids moyen de 21 g par une injection intrapéritonéale de 0,5 $cm^3$ d'une culture de 22 heures, en bouillon Todd Hewitt (pH = 7,8) de la souche de Streptococcus pyogènes A 561 diluée au 1/1000ème par de l'eau physiologique.

On a administré per os une heure, cinq heures, vingt-quatre heures après l'infection une quantité déterminée de produit.

On a noté les mortalités au 5ème jour.
Les résultats ont été les suivants:

| Produits | Doses<br>Souris survivantes au 5ème jour | | |
|---|---|---|---|
| | 0,1 mg | 0,5 mg | 1 mg |
| Produits de l'exemple 1 | | 2 | 10 |
| Produit de l'exemple 5 | 10 | | |

Exemples de compositions pharmaceutiques

On a préparé des comprimés renfermant:
- Produits de l'exemple 31 0,5 g
- Excipient q. s. p. 1 g

(Détail de l'excipient: amidon, talc, stéarate de magnésium).

On a préparé des comprimés renfermant:
- Produit de l'exemple 1 0,2 g
- Excipient q. s. p. 1 g

(Détail de l'excipient: amidon, talc, stéarate de magnésium).

**Revendications pour les Etats contractants BE, CH, DE, FR, GB, IT, LI, LU, NL, SE**

1. Les dérivés de l'érythromycine de formule:

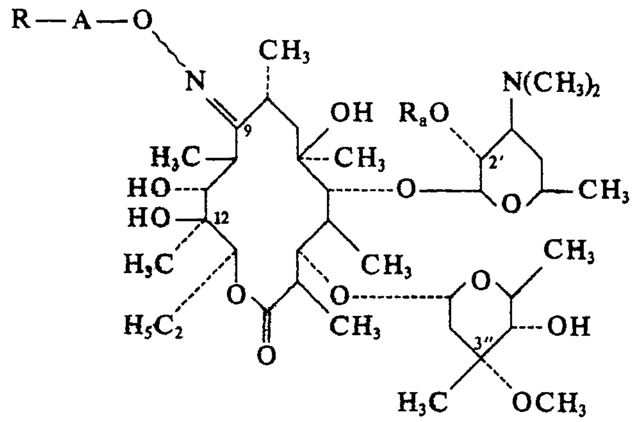

(I)

dans laquelle A représente un radical alkylène linéaire ou ramifié ayant de 1 à 6 atomes de carbone et R représente

- soit un radical alkyloxy, alkényloxy ou alkynyloxy ayant ou plus 6 atomes de carbone,
- soit un radical alkylthio, alkénylthio ou alkynylthio ayant au plus 6 atomes de carbone, l'atome de soufre étant éventuellement oxydé en sulfoxyde ou en sulfone,
- soit un radical aryloxy, aralkyloxy,
- soit un radical arylthio, aralkylthio, l'atome de soufre étant éventuellement oxydé en sulfoxyde ou en sulfone, chacun de ces radicaux étant éventuellement substitué par un ou plusieurs des groupements suivants:
  hydroxy, alkoxy ou alkylthio ayant de 1 à 6 atomes de carbone, vinyloxy, allyloxy, éthynyloxy, propargyloxy, vinylthio, allylthio, éthynylthio, propargylthio, halogène, amino, méthylamino, diméthyl amino, diéthylamino, 1-pipérazinyle, 4-méthyl pipérazin -1-yle, 1-pipéridinyle, 1-pyridinium, N-morpholino, 1-pyrrolyle, pyrrolidinyle, 1-imidazolyle, 1-pyridazinium, 1-pyrimidinium; les radicaux aryloxy, arylthio, aralkyloxy et aralkylthio étant de plus éventuellement substitués par les radicaux méthyle, éthyle, propyle, carbamoyle, aminométhyle, diméthylaminométhyle, aminoéthyle, diméthylaminoéthyle, carboxyle, méthyloxycarbonyle, éthyloxycarbonyle,
- soit R représente un radical

  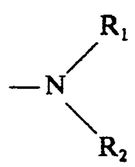

  dans lequel $R_1$ et $R_2$, identiques ou différents, représentent un atome d'hydrogène, un radical alkyle ayant de 1 à 6 atomes de carbone, ou $R_1$ et $R_2$ forment avec l'atome d'azote auquel ils sont liés un hétérocycle choisi dans le groupe formé par 1-pipéridinyle, 1-pipérazinyle, 4-méthyl pipérazin-1-yle, 1-pyridinium, N-morpholinyle, 1-pyrrolyle, 1-imidazolyle, 1-pyridazinium, 1-pyrimidinium,
- soit R représente un groupement ammonium quaternaire,
- soit R représente un atome d'halogène,
- soit R représente un groupement 1,2-époxyéthyle ou 2,2-diméthyl 1,2-époxyéthyle ou un radical résultant de l'ouverture de ce groupement par un réactif nucléophile,

– soit R représente un groupement

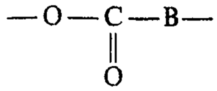

dans lequel B représente soit un radical alkyle ou alkyloxy ayant au plus 6 atomes de carbone

– soit un radical aryle, aralkyle, aryloxy, ou aralkyloxy,

– soit R représente un radical formyle libre ou protégé, un radical carboxyle libre, estérifié ou salifié, un radical thiocyanate, un radical nitrile, acyle ou un radical carbamoyle, Ra représente un atome d'hydrogène ou un radical acyle; le trait ondulé signifie que les produits peuvent se trouver sous la forme syn ou anti ou sous la forme d'un mélange syn et anti;

ainsi que les sels d'addition de ces produits avec les acides minéraux ou organiques.

2. Les produits selon la revendication 1, caractérisés en ce que R représente un groupement alkyloxy, alkényloxy, alkynyloxy, alkylthio, alkénylthio, alkynylthio, aryloxy, aralkyloxy, arylthio ou aralkylthio éventuellement substitué par un ou plusieurs des groupements suivants:

– alkyloxy ou alkylthio ayant de 1 à 6 atomes de carbone,

– halogène,

ou R représente le groupement

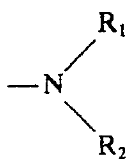

dans lequel $R_1$ et $R_2$ ont la signification indiquée à la revendication 1.

3. Les produits selon la revendication 1, dans laquelle R représente:

soit un radical alkyloxy éventuellement substitué par un radical alkyloxy ou par un radical dialkylamino;
soit un radical aryloxy ou aralkyloxy éventuellement substitué par un atome d'halogène;
soit un radical alkylthio ou arylthio éventuellement substitué par un atome d'halogène;
soit un radical dialkylamino;
soit un atome d'halogène.

4. Les produits selon la revendication 1, de formule (I'):

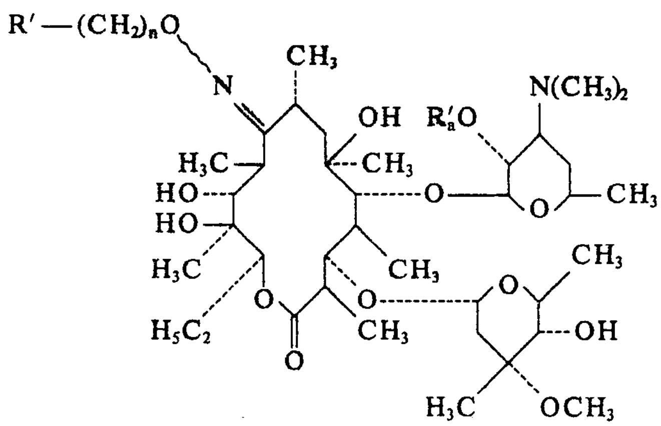

(I')

dans laquelle n représente un entier de 1 à 6 et R' représente

– soit un radical alkyloxy ou alkyloxyalkyloxy ayant au plus 6 atomes de carbone,

– soit un radical phényloxy ou benzyloxy éventuellement substitué par un atome de chlore,

– soit un radical dialkylamino;

R'a représente un atome d'hydrogène ou un radical acyle ayant de 2 à 6 atomes de carbone, ainsi que leurs sels d'addition avec les acides minéraux ou organiques; produits de formule (I') correspondant aux produits de formule (I) dans laquelle A représente un radical alkylène saturé $-(CH_2)_n-$, R a les valeurs de R' et Ra a les valeurs de R'a.

5. Les produits selon la revendication 4, caractérisés en ce que R' représente:

– soit un radical $CH_3-(CH_2)_{n1}-O-$ dans lequel n1 représente un entier de 0 à 3,

– soit un radical $CH_3-(CH_2)_{n1}-O-(CH_2)_{n2}-O-$ dans lequel n1 a la signification précédente et n2 représente un entier de 1 à 3.

6. Les produits selon la revendication 4, caractérisés en ce que R' représente un radical

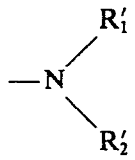

dans lequel $R'_1$ et $R'_2$ représentent un radical alkyle ayant de 1 à 3 atomes de carbone.

7. L'un quelconque des produits de formule I, telle que définie à la revendication 1, dont les noms suivent:

– le 9-[O-[(2-méthoxyéthoxy) méthyl] oxime] de l'érythromycine,
– le 9-[O-[2-(diméthylamino) éthyl] oxime] de l'érythromycine,
– le 9-[O-[2-(diéthylamino) éthyl] oxime] de l'érythromycine,

ainsi que les sels d'addition de ces produits avec les acides minéraux ou organiques.

8. Procédé de préparation des produits de formule (I) telle que définie à la revendication 1, caractérisé en ce que

soit l'on traite éventuellement en présence d'une base, l'érythromycine de formule (II):

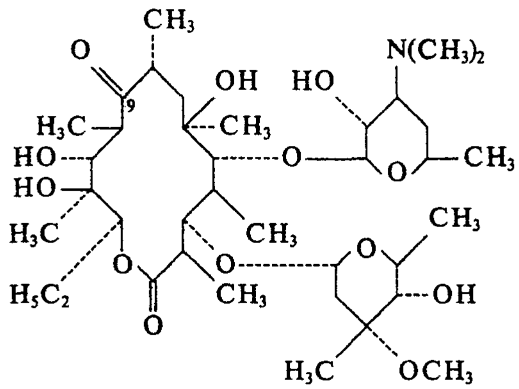

(II)

par un produit de formule (III):

$H_2N–O–A–R$ (III)

dans lequel A et R sont définis comme à la revendication 1, ou par un sel d'acide fort de ce produit, pour obtenir un produit de formule ($I_1$) correspondant à un produit de formule (I) dans laquelle Ra représente un atome d'hydrogène,

soit l'on traite, éventuellement en présence d'une base, la 9-oxime d'érythromycine de formule (IV):

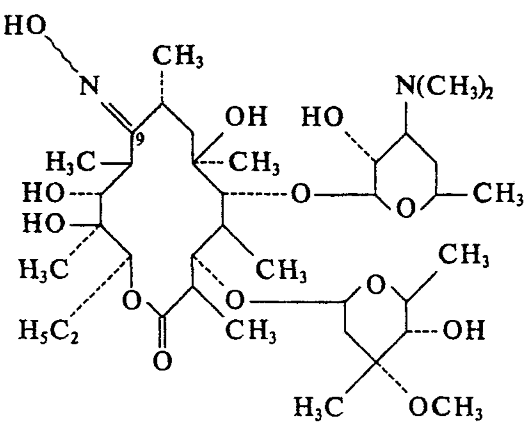

(IV)

sous l'une ou l'autre de ses formes isomères, ou sous forme de mélange, par un produit de formule (V):

Hal–A–R (V)

dans lequel A et R sont définis comme à la revendication 1, et Hal représente un atome d'halogène, pour obtenir un produit de formule ($I_1$) correspondant à un produit de formule (I) dans laquelle Ra représente un atome d'hydrogène, produit de formule ($I_1$) que, le cas échéant,

– dans le cas où R représente un atome d'halogène ou un radical alkoxy, alkényloxy, alkynyloxy, alkylthio, alkénylthio ou alkynylthio substitué par un atome d'halogène, l'on peut également traiter par un réactif de formule

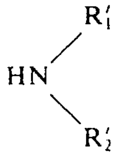

dans lequel $R'_1$ et $R'_2$, identiques ou différents, représentent un atome d'hydrogène ou un radical alkyle linéaire ou ramifié renfermant de 1 à 6 atomes de carbone ou forment avec l'atome d'azote un hétérocycle choisi dans le groupe formé par 1-pipéridinyle, 1-pipérazinyle, 4-méthyl pipérazin-1-yle, 1-pyridinium, N-morpholinyle, 1-pyrrolyle, 1-imidazolyle, 1-pyridazinium, 1-pyrimidinium, pour obtenir un produit correspondant de formule ($I_2$), correspondant à un produit de formule (I) dans laquelle Ra représente un atome d'hydrogène et R représente un radical

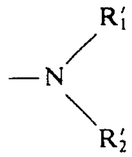

ou un radical alkoxy, alkényloxy, alkynyloxy, alkylthio, alkénylthio ou alkynylthio substitué par un radical

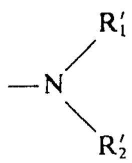 le radical 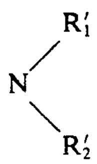

étant tel que défini précédemment,

– dans le cas où R représente un radical 1,2-époxyéthyle éventuellement substitué, l'on peut également traiter par un réactif nucléophile tel que défini précedemment, pour obtenir un produit correspondant de formule ($I_3$), correspondant à un produit de formule (I) dans laquelle Ra représente un atome d'hydrogène et R représente un radical résultant de l'ouverture de l'époxy, produits de formule (I) dans laquelle Ra représente un atome d'hydrogène que, le cas écheant, l'on estérifie pour obtenir des produits de formule (I) dans laquelle Ra représente un radical acyle, et produits de formule (I) que, si désiré, l'on salifie.

9. Procédé de préparation selon la revendication 8, caractérisé en ce que, soit l'on traite un produit de formule (II) par un chlorhydrate ou un bromhydrate d'un produit de formule (III) et en ce que l'one opère en présence d'une base organique aminée ou d'un carbonate de métal alcalino-terreux, soit l'on traite un produit de formule (IV) par un produit de formule (V) en présence d'une base choisie dans le groupe formé par le carbonate et le carbonate acide de sodium ou de potassium, le carbonate de calcium, le carbonate de baryum et l'hydrure de sodium.

10. A titre de médicaments, les produits tels que définis à la revendication 1, pharmaceutiquement acceptables.

11. A titre de médicaments, les produits tels que définis à l'une quelconque des revendications 2 à 6, pharmaceutiquement acceptables.

12. A titre de médicaments, l'un quelconque des produits de formule (I) tels que définis à la revendication 7, pharmaceutiquement acceptables.

13. Les compositions pharmaceutiques contenant, comme principe actif, l'un au moins des médicaments selon l'une quelconque des revendications 10 à 12.

**Revendications pour l'Etat contractant AT**

1. Procédé pour préparer les dérivés de l'érythromycine de formule:

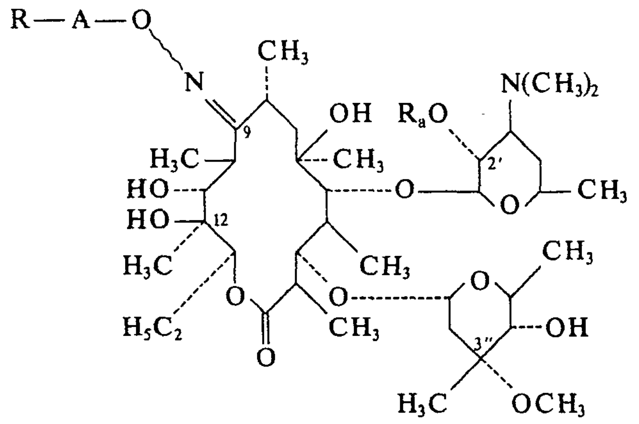

(I)

dans laquelle A représente un radical alkylène linéaire ou ramifié ayant de 1 à 6 atomes de carbone et R représente

- soit un radical alkyloxy, alkényloxy ou alkynyloxy ayant au plus 6 atomes de carbone,
- soit un radical alkylthio, alkénylthio ou alkynylthio ayant au plus 6 atomes de carbone, l'atome de soufre étant éventuellement oxydé en sulfoxyde ou en sulfone,
- soit un radical aryloxy, aralkyloxy,
- soit un radical arylthio, aralkylthio, l'atome de soufre étant éventuellement oxydé en sulfoxyde ou en sulfone, chacun de ces radicaux étant éventuellement substitué par un ou plusieurs des groupements suivants:
  hydroxy, alkoxy ou alkylthio ayant de 1 à 6 atomes de carbone, vinyloxy, allyloxy, éthynyloxy, propargyloxy, vinylthio, allylthio, éthynylthio, propargylthio, halogène, amino, méthylamino, diméthyl amino, diéthylamino, 1-pipérazinyle, 4-méthyl pipérazin-1-yle, 1-pipéridinyle, 1-pyridinium, N-morpholino, 1-pyrrolyle, pyrrolidinyle, 1-imidazolyle, 1-pyridazinium, 1-pyrimidinium; les radicaux aryloxy, arylthio, aralkyloxy et aralkylthio étant de plus éventuellement substitués par les radicaux méthyle, éthyle, propyle, carbamoyle, aminométhyle, diméthylaminométhyle, aminoéthyle, diméthylaminoéthyle, carboxyle, méthyloxycarbonyle, éthyloxycarbonyle,
- soit R représente un radical

  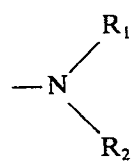

  dans lequel $R_1$ et $R_2$, identiques ou différents, représentent un atome d'hydrogène, un radical alkyle ayant de 1 à 6 atomes de carbone, ou $R_1$ et $R_2$ forment avec l'atome d'azote auquel ils sont liés un hétérocycle choisi dans le groupe formé par 1-pipéridinyle, 1-pipérazinyle, 4-méthyl pipérazin-1-yle, 1-pyridinium, N-morpholinyle, 1-pyrrolyle, 1-imidazolyle, 1-pyridazinium, 1-pyrimidinium,
- soit R représente un groupement ammonium quaternaire,
- soit R représente un atome d'halogène,
- soit R représente un groupement 1,2-époxyéthyle ou 2,2-diméthyl 1,2-époxyéthyle ou un radical résultant de l'ouverture de ce groupement par un réactif nucléophile,
- soit R représente un groupement

  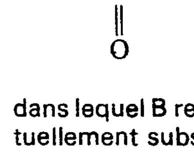

  dans lequel B représente soit un radical alkyle ou alkyloxy ayant au plus 6 atomes de carbone éventuellement substitué, soit un radical aryle, aralkyle, aryloxy, ou aralkyloxy,
- soit R représente un radical formyle libre ou protégé, un radical carboxyle libre, estérifié ou salifié, un radical thiocyanate, un radical nitrile, acyle ou un radical carbamoyle, Ra représente un atome d'hydrogène ou un radical acyle; le trait ondulé signifie que les produits peuvent se trouver sous la forme syn ou anti ou sous le forme d'un mélange syn et anti;

ainsi que les sels d'addition de ces produits avec les acides minéraux ou organiques, caractérisé en ce que:

soit l'on traite, éventuellement en présence d'une base, l'erythromycine de formule (II):

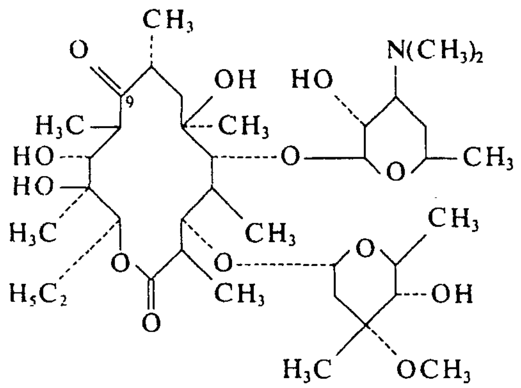

(II)

par un produit de formule (III):

$H_2N{-}O{-}A{-}R$ (III)

dans lequel A et R sont définis comme précédemment, ou par un sel d'acide fort de ce produit, pour obtenir un produit de formule ($I_1$) correspondant à un produit de formule (I) dans laquelle Ra représente un atome d'hydrogène,
soit l'on traite, éventuellement en présence d'une base, la 9-oxime d'érythromycine de formule (IV):

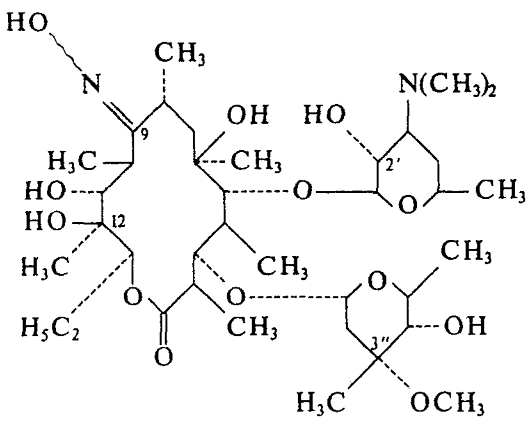

(IV)

sous l'une ou l'autre de ses formes isomères, ou sous forme de mélange, par un produit de formule (V):

Hal—A—R (V)

dans lequel A et R sont définis comme précédemment et Hál représente un atome d'halogène, pour obtenir un produit de formule ($I_1$) correspondant à un produit de formule (I) dans laquelle Ra représente un atome d'hydrogène, produit de formule ($I_1$) que, le cas échéant,

— dans le cas où R représente un atome d'halogène ou un radical alkoxy, alkényloxy, alkynyloxy, alkylthio, alkénylthio ou alkylnylthio substitué par un atome d'halogène, l'on peut également traiter par un réactif de formule

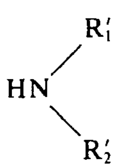

dans lequel $R'_1$ et $R'_2$, identiques ou différents, représentent un atome d'hydrogène ou un radical alkyle linéaire ou ramifié renfermant de 1 à 6 atomes de carbone ou forment avec l'atome d'azote un hétérocycle renfermant éventuellement un autre hétéroatome et éventuellement substitué, pour obtenir un produit correspondant de formule ($I_2$), correspondant à un produit de formule (I) dans laquelle Ra représente un atome d'hydrogène et R représente un radical

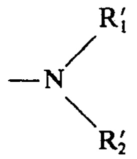

ou un radical alkoxy, alkényloxy, alkynyloxy, alkylthio, alkénylthio ou alkynylthio substitué par un radical.

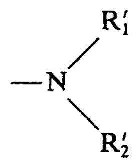 le radical 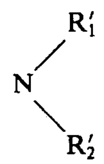

étant tel que défini précédemment,

– dans le cas où R représente un radical 1,2-époxyéthyle éventuellement substitué, l'on peut également traiter par un réactif nucléophile tel que défini précédemment, pour obtenir un produit correspondant de formule ($I_3$), correspondant à un produit de formule (I) dans laquelle Ra représente un atome d'hydrogène et R représente un radical résultant de l'ouverture de l'époxy, produits de formule (I) dans laquelle Ra représente un atome d'hydrogène que, le cas échéant, l'on estérifie pour obtenir des produits de formule (I) dans laquelle Ra représente un radical acyle, et produits de formule (I) que, si désiré, l'on salifie.

2. Procédé de préparation selon la revendication 1, caractérisé en ce que, soit l'on traite un produit de formule (II) par un chlorhydrate ou un bromhydrate d'un produit de formule (III) et en ce que l'on opère en présence d'une base organique aminée ou d'un carbonate de métal alcalino-terreux, soit l'on traite un produit de formule (IV) par un produit de formule (V) en présence d'une base choisie dans le groupe formé par le carbonate et le carbonate acide de sodium ou de potassium, le carbonate de calcium, le carbonate de baryum et l'hydrure de sodium.

3. Procédé selon la revendication 1 ou 2 caractérisé en ce que l'on utilise au départ un produit de formule III ou V dans lequel R représente un groupement alkyloxy, alkényloxy, alkynyloxy, alkylthio, alkénylthio, alkynylthio, aryloxy, aralkyloxy, arylthio ou aralkylthio éventuellement substitué par un ou plusieurs des groupements suivants:

– alkyloxy ou alkylthio ayant de 1 à 6 atomes de carbone,
– halogène,

ou R représente le groupement

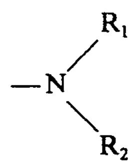

dans lequel $R_1$ et $R_2$ ont la signification indiquée à la revendication 2.

4. Procédé selon la revendication 1 ou 2 caractérisé en ce que l'on utilise au départ un produit de formule III or V dans lequel R représente:

soit un radical alkyloxy éventuellement substitué par un radical alkyloxy ou par un radical dialkylamino;
soit un radical aryloxy ou aralkyloxy éventuellement substitué par un atome d'halogène;
soit un radical alkylthio ou arylthio éventuellement substitué par un atome d'halogène;
soit un radical dialkylamino;
soit un atome d'halogène.

5. Procédé selon la revendication 1 ou 2 caractérisé en ce que l'on utilise au départ un produit de formule III ou V dans lequel R représente:

– soit un radical alkyloxy ou alkyloxyalkyloxy ayant au plus 6 atomes de carbone,
– soit un radical phényloxy ou benzyloxy éventuellement substitué par un atome de chlore,
– soit un radical dialkylamino et A représente un radical alkylène saturé $-(CH_2)_n-$.

6. Procédé selon la revendication 1 ou 2 caractérisé en ce que l'on utilise au départ un produit de formule III ou V dans lequel R représente:

– soit un radical $CH_3-(CH_2)_{n1}-O-$ dans lequel n1 représente un entier de 0 à 3,
– soit un radical $CH_3-(CH_2)_{n1}-O-(CH_2)_{n2}-O-$dans lequel n1 a la signification précédente et n2 représente un entier de 1 à 3.

7. Procédé selon la revendication 1 ou 2 caractérisé en ce que l'on utilise au départ un produit de formule III ou V dans lequel R représente un radical

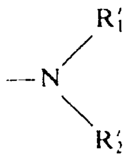

dans lequel $R'_1$ et $R'_2$ représentent un radical alkyle ayant de 1 à 3 atomes de carbone.

8. Procédé selon la revendication 1 ou 2, caractérisé en ce que l'on utilise au départ un produit de formule III ou V choisi de manière telle que l'on prépare l'un quelconque des produits de formule I, telle que définie à la revendication 1, dont les noms suivent:

– le 9-[0-[(2-méthoxyéthoxy) méthyl] oxime] de l'érythromycine,
– le 9-[0-[2-(diméthylamino) éthyl] oxime] de l'érythromycine,
– le 9-[0-[2-(diéthylamino) éthyl] oxime] de l'érythromycine,

ainsi que les sels d'addition de ces produits avec les acides minéraux ou organiques.

**Patentansprüche für die Vertragsstaaten: BE, CH, DE, FR, GB, IT, LI, LU, NL, SE**

1. Erythromycinderivate der Formel

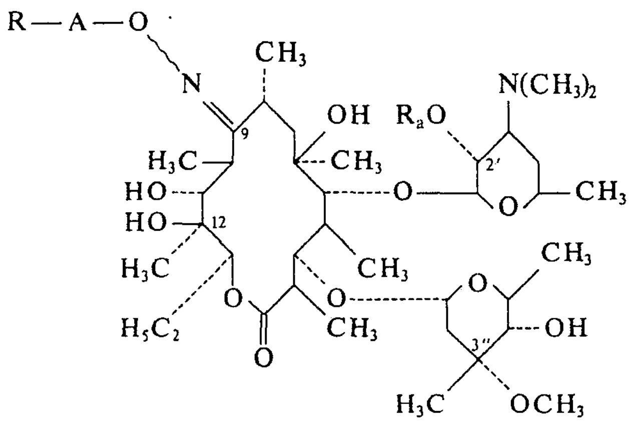

(I)

worin A eine linearen oder verzweigten Alkylenrest mit 1 bis 6 Kohlenstoffatomen bedeutet und R bedeutet:

entweder einen Alkyloxy-, Alkenyloxy- oder Alkinyloxyrest mit höchstens 6 Kohlenstoffatomen, oder einen Alkylthio-, Alkenylthio- oder Alkinylthiorest mit höchstens 6 Kohlenstoffatomen, wobei das Schwefelatom gegebenenfalls zum Sulfoxid oder zum Sulfon oxidiert ist,
oder einen Aryloxy- oder Aralkyloxyrest,
oder einen Arylthio-, Aralkylthiorest, wobei das Schwefelatom gegebenenfalls zum Sulfoxid oder zum Sulfon oxidiert ist und wobei ein jeder dieser Reste gegebenenfalls durch eine oder mehrere der folgenden Gruppen substituiert ist:

Hydroxy, Alkoxy oder Alkylthio mit 1 bis 6 Kohlenstoffatomen, Vinyloxy, Allyloxy, Ethinyloxy, Propargyloxy, Vinylthio, Allylthio, Ethinylthio, Propargylthio, Halogen, Amino, Methylamino, Dimethylamino, Diethylamino, 1-Piperazinyl, 4-Methyl-piperazin-1-yl, 1-Piperidinyl, 1-Pyridinium, N-Morpholino, 1-Pyrrolyl, Pyrrolidinyl, 1-Imidazolyl, 1-Pyridazinium, 1-Pyrimidinium; wobei die Aryloxy-, Arylthio-, Aralkyloxy- und Aralkylthioreste außerdem gegebenenfalls substituiert sind durch Methyl-, Ethyl-, Propyl-, Carbamoyl-, Aminomethyl-, Dimethylaminomethyl-, Aminoethyl-, Dimethylaminoethyl-, Carboxyl-, Methyloxycarbonyl-, Ethyloxycarbonylreste,

oder R einen Rest

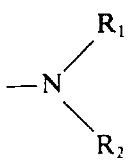

bedeutet, worin $R_1$ und $R_2$, die gleich oder verschieden sein können, ein Wasserstoffatom oder einen Alkylrest mit 1 bis 6 Kohlenstoffatomen bedeuten, oder $R_1$ und $R_2$ mit dem Stickstoffatom, an das sie gebunden sind, einen Heterocyclus bilden, ausgewählt unter 1-Piperidinyl, 1-Piperazinyl, 4-Methylpiperazin-1-yl, 1-Pyridinium, N-Morpholinyl, 1-Pyrrolyl, 1-Imidazolyl, 1-Pyridazinium, 1-Pyrimidinium, oder R eine quaternäre Ammoniumgruppe bedeutet,

oder R ein Halogenatom bedeutet,
oder R eine 1,2-Epoxyethyl- oder 2,2-Dimethyl-1,2-epoxyethylgruppe oder einen Rest, entstammend der Öffnung dieser Gruppe durch ein nukleophiles Reagens, bedeutet,
oder R eine Gruppe

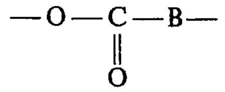

bedeutet, worin B entweder einen Alkyl- oder Alkyloxyrest mit höchstens 6 Kohlenstoffatomen oder einen Aryl-, Aralkyl-, Aryloxy- oder Aralkyloxyrest bedeutet,
oder R einen freien oder geschützten Formylrest, einen freien, veresterten oder in ein Salz übergeführten Carboxylrest, einen Thiocyanatrest, einen Nitrilrest, einen Acylrest oder einen Carbamoylrest bedeutet,
Ra ein Wasserstoffatom oder einen Acylrest bedeutet, wobei die gewellte Linie anzeigt, daß die Produkte sich in der syn- oder anti-Form oder in Form eines Gemisches der syn- und anti-Form befinden können;
sowie die Additionssalze dieser Produkte mit Mineral- oder organischen Säuren.

2. Produkte gemäß Anspruch 1, dadurch gekennzeichnet, daß R eine Alkyloxy-, Alkenyloxy-, Alkinyloxy-, Alkylthio-, Alkenylthio-, Alkinylthio-, Aryloxy-, Aralkyloxy-, Arylthio- oder Aralkylthiogruppe bedeutet, die gegebenenfalls substituiert ist durch eine oder mehrere der folgenden Gruppen:

Alkyloxy oder Alkylthio mit 1 bis 6 Kohlenstoffatomen,
Halogen,
oder R die Gruppe

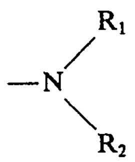

bedeutet, worin $R_1$ und $R_2$ die in Anspruch 1 angegebene Bedeutung besitzen.

3. Produkte gemäß Anspruch 1, worin R bedeutet:

entweder einen gegebenenfalls durch einen Alkyloxyrest oder durch einen Dialkylaminorest substituierten Alkyloxyrest;
oder einen gegebenenfalls durch ein Halogenatom substituierten Aryloxy- oder Aralkyloxyrest;
oder einen gegebenenfalls durch ein Halogenatom substituierten Alkylthio- oder Arylthiorest;
oder einen Dialkylaminorest;
oder ein Halogenatom.

4. Produkte gemäß Anspruch 1 der Formel (I')

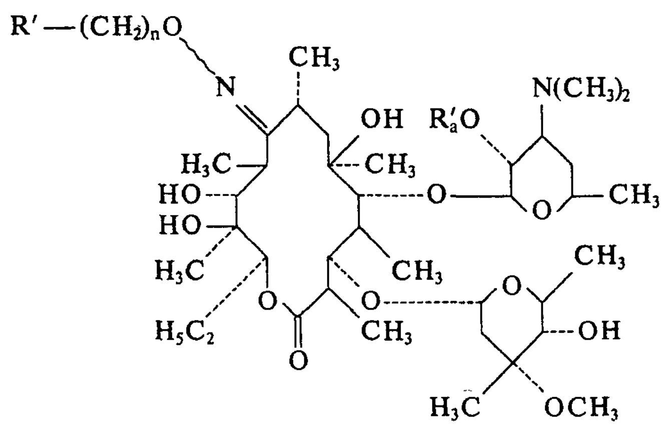

(I')

worin n eine ganze Zahl von 1 bis 6 bedeutet und R' bedeutet:

entweder einen Alkyloxy- oder Alkyloxyalkyloxyrest mit höchstens 6 Kohlenstoffatomen,
oder einen gegebenenfalls durch ein Chloratom substituierten Phenyloxy- oder Benzyloxyrest,

oder einen Dialkylaminorest;
R'a ein Wasserstoffatom oder einen Acylrest mit 2 bis 6 Kohlenstoffatomen bedeutet, sowie deren Additionssalze mit Mineral- oder organischen Säuren, wobei die Produkte der Formel (I') den Produkten der Formel (I) entsprechen, worin A einen gesättigten Alkylenrest $-(CH_2)_n-$ bedeutet, R die Bedeutungen von R' hat und Ra die Bedeutungen von R'a hat.

5. Produkte gemäß Anspruch 4, dadurch gekennzeichnet, daß R' bedeutet:

entweder einen Rest $CH_3-(CH_2)_{n1}-O-$, worin n1 eine ganze Zahl von 0 bis 3 ist,
oder einen Rest $CH_3-(CH_2)_{n1}-O-(CH_2)_{n2}-O-$, worin n1 die vorstehende Bedeutung besitzt und n2 eine ganze Zahl von 1 bis 3 bedeutet.

6. Produkte gemäß Anspruch 4, dadurch gekennzeichnet, daß R' einen Rest

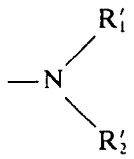

darstellt, worin $R'_1$ und $R'_2$ einen Alkylrest mit 1 bis 3 Kohlenstoffatomen bedeuten.

7. Eines der Produkte der Formel I gemäß Anspruch 1 mit den folgenden Bezeichnungen:

9-[O-[(2-Methoxyethoxy)-methyl]-oxim] von Erythromycin,
9-[O-[2-(Dimethylamino)-ethyl]-oxim] von Erythromycin,
9-[O-[2-(Diethylamino)-ethyl]-oxim] von Erythromycin

sowie die Additionssalze dieser Produkte mit Mineral- und organischen Säuren.

8. Verfahren zur Herstellung der Produkte der Formel (I) gemäß Anspruch 1, dadurch gekennzeichnet, daß man

entweder gegebenenfalls in Gegenwart einer Base Erythromycin der Formel (II)

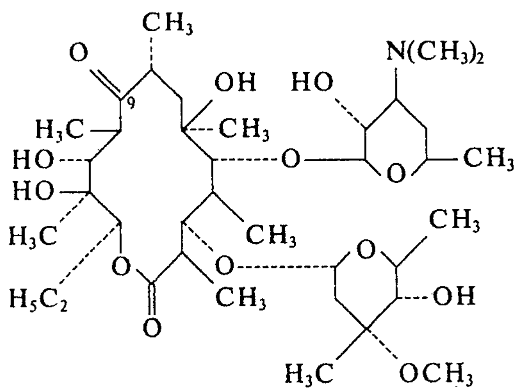

(II)

mit einem Produkt der Formel (III):

$$H_2N-O-A-R \qquad (III)$$

worin A und R wie in Anspruch 1 definiert sind, oder mit einem Salz einer starken Säure dieses Produktes behandelt, um ein Produkt der Formel ($I_1$) entsprechend einem Produkt der Formel (I), worin Ra ein Wasserstoffatom bedeutet, zu erhalten,

oder gegebenenfalls in Gegenwart einer Base das Erythromycin-9-oxim der Formel (IV):

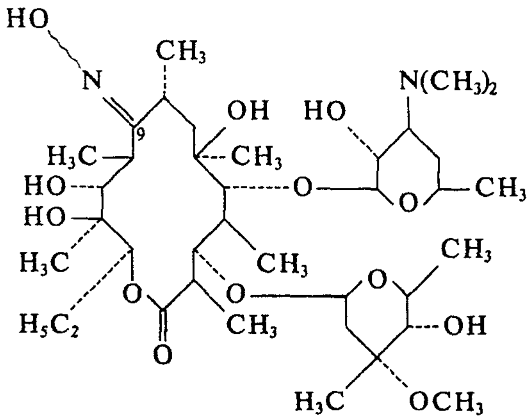

(IV)

in der einen oder anderen Form seiner Isomeren oder in Form eines Gemisches derselben mit einem Produkt der Formel (V):

Hal—A—R (V)

worin A und R wie in Anspruch 1 definiert sind und Hal ein Halogenatom bedeutet, behandelt, um ein Produkt der Formel $(I_1)$ entsprechend einem Produkt der Formel (I), worin Ra ein Wasserstoffatom bedeutet, zu erhalten, welches Produkt der Formel $(I_1)$ man gegebenenfalls
wenn R ein Halogenatom oder einen Alkoxy-, Alkenyloxy-, Alkinyloxy-, Alkylthio-, Alkenylthio- oder Alkinylthiorest, der durch ein Halogenatom substituiert ist, bedeutet, auch mit einem Reagens der Formel

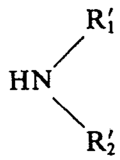

worin $R'_1$ und $R'_2$, die gleich oder verschieden sein können, ein Wasserstoffatom oder einen linearen oder verzweigten Alkylrest mit 1 bis 6 Kohlenstoffatomen bedeuten, oder mit dem Stickstoffatom einen Heterocyclus bilden, ausgewählt unter 1-Piperidinyl, 1-Piperazinyl, 4-Methylpiperazin-1-yl, 1-Pyridinium, N-Morpholinyl, 1-Pyrrolyl, 1-Imidazolyl, 1-Pyridazinium, 1-Pyrimidinium, behandeln kann, um ein entsprechendes Produkt der Formel $(I_2)$, entsprechend einem Produkt der Formel (I), worin Ra ein Wasserstoffatom bedeutet und R einen Rest

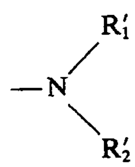

oder einen Alkoxy-, Alkenyloxy-, Alkinyloxy-, Alkylthio-, Alkenylthio- oder Alkinylthiorest, der durch einen Rest

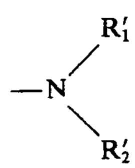

substituiert ist, darstellt, zu erhalten, wobei der Rest

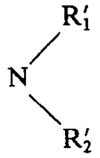

wie vorstehend definiert ist, wenn R einen 1,2-Epoxyethylrest, der gegebenenfalls substituiert ist, bedeutet, auch mit einem nukleophilen Reagens, wie vorstehend definiert, behandeln kann, um ein entsprechendes Produkt der Formel ($I_3$), entsprechend einem Produkt der Formel (I), worin Ra ein Wasserstoffatom bedeutet und R einen der Öffnung der Epoxygruppe entstammenden Rest darstellt, zu erhalten, die Produkte der Formel (I), worin Ra ein Wasserstoffatom bedeutet, gegebenenfalls verestert, um Produkte der Formel (I) zu erhalten, worin Ra einen Acylrest darstellt, und die Produkte der Formel (I) gewünschtenfalls in ein Salz überführt.

9. Verfahren gemäß Anspruch 8, dadurch gekennzeichnet, daß man entweder ein Produkt der Formel (II) mit einem Hydrochlorid oder einem Hydrobromid eines Produkts der Formel (III) behandelt und daß man in Gegenwart einer organischen Aminbase oder eines Erdalkalimetallcarbonats arbeitet oder ein Produkt der Formel (IV) mit einem Produkt der Formel (V) in Gegenwart einer Base, ausgewählt unter Natrium- oder Kaliumcarbonat und -bicarbonat, Calciumcarbonat, Bariumcarbonat und Natriumhydrid behandelt.

10. Als Arzneimittel die pharmazeutisch verträglichen Produkte gemäß Anspruch 1.

11. Als Arzneimittel die pharmazeutisch verträglichen Produkte gemäß einem der Ansprüche 2 bis 6.

12. Als Arzneimittel eines der pharmazeutisch verträglichen Produkte der Formel (I) gemäß Anspruch 7.

13. Pharmazeutische Zusammensetzungen, enthaltend als Wirkstoff zumindest eines der Arzneimittel gemäß einem der Ansprüche 10 bis 12.

**Patentansprüche für den Vertragsstaat: AT**

1. Verfahren zur Herstellung von Erythromycinderivaten der Formel:

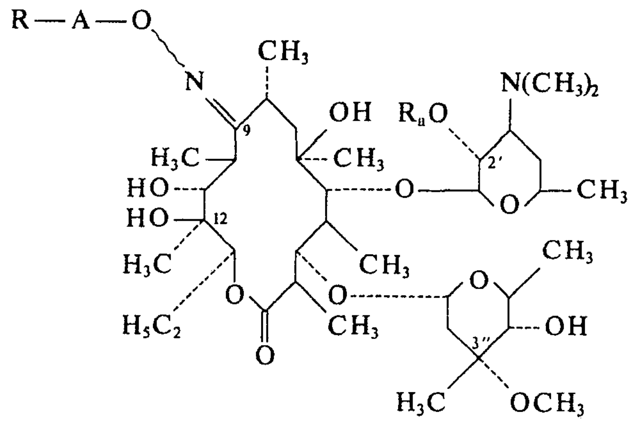

(I)

worin A einen linearen oder verzweigten Alkylenrest mit 1 bis 6 Kohlenstoffatomen bedeutet und R bedeutet:

entweder einen Alkyloxy-, Alkenyloxy- oder Alkinyloxyrest mit höchstens 6 Kohlenstoffatomen, oder einen Alkylthio-, Alkenylthio- oder Alkinylthiorest mit höchstens 6 Kohlenstoffatomen, wobei das Schwefelatom gegebenenfalls zum Sulfoxid oder zum Sulfon oxidiert ist, oder einen Aryloxy- oder Aralkyloxyrest, oder einen Arylthio-, Aralkylthiorest, wobei das Schwefelatom gegebenenfalls zum Sulfoxid oder zum Sulfon oxidiert ist und wobei ein jeder dieser Reste gegebenenfalls durch eine oder mehrere der folgenden Gruppen substituiert ist:

Hydroxy, Alkoxy oder Alkylthio mit 1 bis 6 Kohlenstoffatomen, Vinyloxy, Allyloxy, Ethinyloxy, Propargyloxy, Vinylthio, Allylthio, Ethinylthio, Propargylthio, Halogen, Amino, Methylamino, Dimethylamino, Diethylamino, 1-Piperazinyl, 4-Methyl-piperazin-1-yl, 1-Piperidinyl, 1-Pyridinium, N-Morpholino, 1-Pyrrolyl, Pyrrolidinyl, 1-Imidazolyl, 1-Pyridazinium, 1-Pyrimidinium; wobei die Aryloxy-, Arylthio-, Aralkyloxy- und Aralkylthioreste außerdem gegebenenfalls substituiert sind durch Methyl-, Ethyl-, Propyl-, Carbamoyl-, Aminomethyl-, Dimethylaminomethyl-, Aminoethyl-, Dimethylaminoethyl-, Carboxyl-, Methyloxycarbonyl-, Ethyloxycarbonylreste,

oder R einen Rest

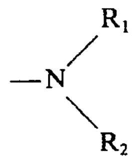

bedeutet, worin $R_1$ und $R_2$, die gleich oder verschieden sind, ein Wasserstoffatom oder einen Alkylrest mit 1 bis 6 Kohlenstoffatomen bedeuten, oder $R_1$ und $R_2$ mit dem Stickstoffatom, an das sie gebunden sind, einen Heterocyclus bilden, ausgewählt unter 1-Piperidinyl, 1-Piperazinyl, 4-Methylpiperazin-1-yl, 1-Pyridinium, N-Morpholinyl, 1-Pyrrolyl, 1-Imidazolyl, 1-Pyridazinium, 1-Pyrimidinium,
oder R eine quaternäre Ammoniumgruppe bedeutet,
oder R ein Halogenatom bedeutet,
oder R eine 1,2-Epoxyethyl- oder 2,2-Dimethyl-1,2-epoxyethylgruppe oder einen der Öffnung dieser Gruppe durch ein nukleophiles Reagens entstammenden Rest darstellt,
oder R eine Gruppe

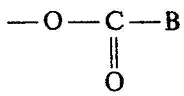

bedeutet, worin B entweder einen Alkyl- oder Alkyloxyrest mit höchstens 6 Kohlenstoffatomen, der gegebenenfalls substituiert ist, oder einen Aryl-, Aralkyl-, Aryloxy- oder Aralkyloxyrest bedeutet,
oder R einen freien oder geschützten Formylrest, einen freien, veresterten oder in ein Salz übergeführten Carboxylrest, einen Thiocyanatrest, einen Nitril- oder Acylrest oder einen Carbamoylrest bedeutet;
Ra ein Wasserstoffatom oder einen Acylrest bedeutet, wobei die gewellte Linie anzeigt, daß die Produkte sich in der syn- oder anti-Form oder in Form eines Gemisches der syn- und anti-Form befinden können;
sowie die Additionssalze dieser Produkte mit Mineral- oder organischen Säuren,

dadurch gekennzeichnet, daß man

entweder gegebenenfalls in Gegenwart einer Base Erythromycin der Formel (II)

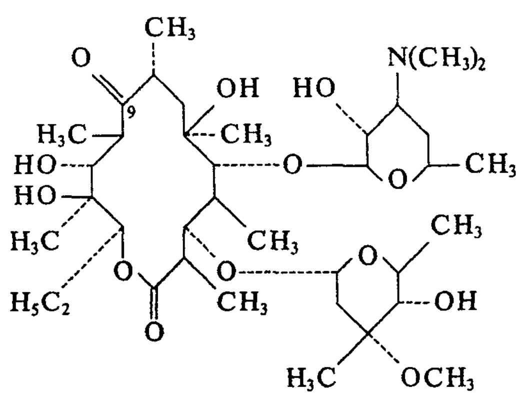

(II)

mit einem Produkt der Formel (III):

$H_2N{-}O{-}A{-}R$ (III)

worin A und R wie vorstehend definiert sind, oder mit einem Salz einer starken Säure dieses Produkts behandelt, um ein Produkt der Formel ($I_1$), entsprechend einem Produkt der Formel (I), worin Ra ein Wasserstoffatom bedeutet, zu erhalten,

oder gegebenenfalls in Gegenwart einer Base das 9-Erythromycin-oxim der Formel (IV):

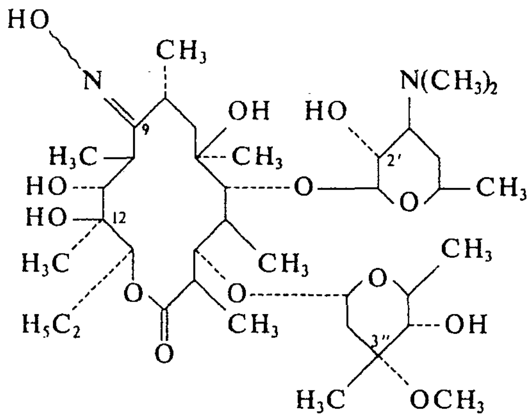

(IV)

in der einen oder anderen seiner isomeren Formen oder in Form eines Gemisches mit einem Produkt der Formel (V):

Hal—A—R (V)

worin A und R wie vorstehend definiert sind und Hal ein Halogenatom bedeutet, behandelt, um ein Produkt der Formel ($I_1$), entsprechend einem Produkt der Formel (I), worin Ra ein Wasserstoffatom bedeutet, zu erhalten, welches Produkt der Formel ($I_1$) man gegebenenfalls

wenn R ein Halogenatom oder einen Alkoxy-, Alkenyloxy-, Alkinyloxy-, Alkylthio-, Alkenylthio- oder Alkinylthiorest, der durch ein Halogenatom substituiert ist, bedeutet, auch mit einem Reagens der Formel

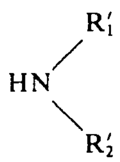

worin $R'_1$ und $R'_2$, die gleich oder verschieden sein können, ein Wasserstoffatom oder einen linearen oder verzweigten Alkylrest mit 1 bis 6 Kohlenstoffatomen bedeuten oder mit dem Stickstoffatom einen gegebenenfalls ein weiteres Heteroatom enthaltenden Heterocyclus, der gegebenenfalls substituiert ist, bilden, behandeln kann, um ein entsprechendes Produkt der Formel ($I_2$), entsprechend einem Produkt der Formel (I), worin Ra ein Wasserstoffatom bedeutet und R einen Rest

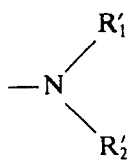

oder einen Alkoxy-, Alkenyloxy-, Alkinyloxy-, Alkylthio-, Alkenylthio- oder Alkinylthiorest, der durch einen Rest

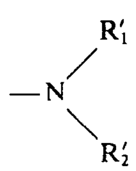

substituiert ist, bedeutet, wobei der Rest

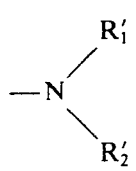

wie vorstehend definiert ist, zu erhalten,

oder, wenn R einen 1,2-Epoxyethylrest, der gegebenenfalls substituiert ist, bedeutet, auch mit einem nukleophilen Rest, wie vorstehend definiert, behandeln kann, um ein entsprechendes Produkt der Formel ($I_3$), entsprechend einem Produkt der Formel (I), worin Ra ein Wasserstoffatom bedeutet und R einen der Öffnung der Epoxygruppe entstammenden Rest bedeutet, zu erhalten, wobei man die Produkte der Formel (I), worin Ra ein Wasserstoffatom bedeutet, gegebenenfalls verestert, um Produkte der Formel (I) zu erhalten, worin Ra einen Acylrest bedeutet, und die Produkte der Formel (I) gegebenenfalls in ein Salz überführt.

2. Verfahren gemäß Anspruch 1, dadurch gekennzeichnet, daß man entweder ein Produkt der Formel (II) mit einem Hydrochlorid oder einem Hydrobromid eines Produkts der Formel (III) behandelt und daß man in Gegenwart einer organischen Aminbase oder eines Erdalkalimetallcarbonats arbeitet, oder ein Produkt der Formel (IV) mit einem Produkt der Formel (V) in Gegenwart einer Base, ausgewählt unter Natrium- oder Kaliumcarbonat und -bicarbonat, Calciumcarbonat, Bariumcarbonat und Natriumhydrid, behandelt.

3. Verfahren gemäß Anspruch 1 oder 2, dadurch gekennzeichnet, daß man von einem Produkt der Formel (III) oder (V) ausgeht, worin R eine Alkyloxy-, Alkenyloxy-, Alkinyloxy-, Alkylthio-, Alkenylthio-, Alkinylthio-, Aryloxy-, Aralkyloxy-, Arylthio- oder Aralkylthiogruppe bedeutet, die gegebenenfalls substituiert sein kann durch eine oder mehrere der folgenden Gruppen:

Alkyloxy oder Alkylthio mit 1 bis 6 Kohlenstoffatomen,
Halogen,
oder R die Gruppe

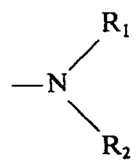

bedeutet, worin $R_1$ und $R_2$ die in Anspruch 2 angegebene Bedeutung besitzen.

4. Verfahren gemäß Anspruch 1 oder 2, dadurch gekennzeichnet, daß man von einem Produkt der Formel (III) oder (V) ausgeht, worin R bedeutet:

entweder einen gegebenenfalls durch einen Alkyloxyrest oder durch einen Dialkylaminorest substituierten Alkyloxyrest;
oder einen gegebenenfalls durch ein Halogenatom substituierten Aryloxy- oder Aralkyloxyrest;
oder einen gegebenenfalls durch ein Halogenatom substituierten Alkylthio- oder Arylthiorest;
oder einen Dialkylaminorest;
oder ein Halogenatom.

5. Verfahren gemäß Anspruch 1 oder 2, dadurch gekennzeichnet, daß man von einem Produkt der Formel (III) oder (V) ausgeht, worin R bedeutet:

entweder einen Alkyloxy- oder Alkyloxyalkyloxyrest mit höchstens 6 Kohlenstoffatomen,
oder einen gegebenenfalls durch ein Chloratom substituierten Phenyloxy- oder Benzyloxyrest,
oder einen Dialkylaminorest und A einen gesättigten Alkylenrest $-(CH_2)n-$ bedeutet.

6. Verfahren gemäß Anspruch 1 oder 2, dadurch gekennzeichnet, daß man von einem Produkt der Formel (III) oder (V) ausgeht, worin R bedeutet:

entweder einen Rest $CH_3-(CH_2)_{n1}-O-$, worin n1 eine ganze Zahl von 0 bis 3 bedeutet,
oder einen Rest $CH_3-(CH_2)_{n1}-O-(CH_2)_{n2}-O-$, worin n1 die vorstehende Bedeutung besitzt und n2 eine ganze Zahl von 1 bis 3 bedeutet.

7. Verfahren gemäß Anspruch 1 oder 2, dadurch gekennzeichnet, daß man von einem Produkt der Formel (III) oder (V) ausgeht, worin R einen Rest

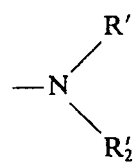

bedeutet, worin $R'_1$ und $R'_2$ einen Alkylrest mit 1 bis 3 Kohlenstoffatomen bedeuten.

8. Verfahren gemäß Anspruch 1 oder 2, dadurch gekennzeichnet, daß man von einem Produkt der Formel (III) oder (V) ausgeht, das derart ausgewählt ist, daß man eines der Produkte der Formel (I), wie in

Anspruch 1 definiert, mit den folgenden Bezeichnungen herstellt:

9-[O-[(2-Methoxyethoxy)-methyl]-oxim] von Erythromycin,
9-[O-[2-(Dimethylamino)-ethyl]-oxim] von Erythromycin,
9-[O-[2-(Diethylamino)-ethyl]-oxim von Erythromycin

sowie die Additionssalze dieser Produkte mit Mineral- oder organischen Säuren.

**Claims for the contracting States: BE, CH, DE, FR, GB, IT, LI, LU, NL, SE**

1. The derivatives of erythromycin with the formula:

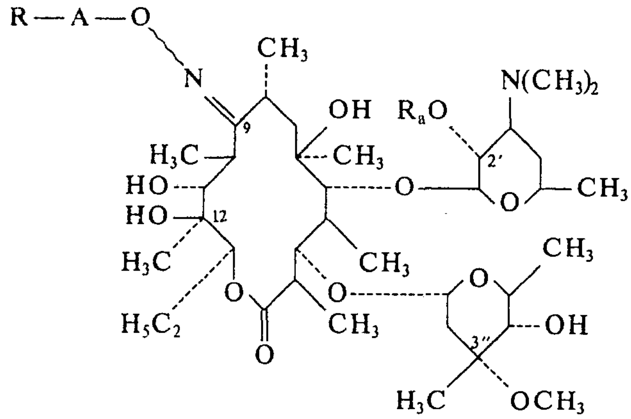

(I)

in which A represents an alkylene radical, linear or branched, with 1—6 carbon atoms, and R represents

- either an alkyloxy, alkenyloxy or alkynyloxy radical with at most 6 carbon atoms,
- or an alkylthio, alkenylthio or alkynylthio radical with at most 6 carbon atoms, the sulphur atom possibly being oxidized to sulphoxide or to sulphone,
- or an aryloxy, aralkyloxy radical,
- or an arylthio, aralkylthio radical, the sulphur atom possibly being oxidized to sulphoxide or to sulphone, each of these radicals being possibly substituted by one or more of the following groups: hydroxy, alkoxy or alkylthio with 1—6 carbon atoms, vinyloxy, allyloxy, ethynyloxy, propargyloxy, vinylthio, allylthio, ethynylthio, propargylthio, halogen, amino, methylamino, dimethylamino, diethylamino, 1-piperazinyl, 4-methyl-piperazin-1-yl, 1-piperidinyl, 1-pyridinium, N-morpholino, 1-pyrrolyl, pyrrolidinyl, 1-imidazolyl, 1-pyridazinium, 1-pyrimidinium; the radicals: aryloxy, arylthio, aralkyloxy and aralkylthio, being moreover, possibly substituted by methyl, ethyl, propyl, carbamoyl, aminomethyl, dimethylaminomethyl, aminoethyl, dimethylaminoethyl, carboxyl, methyloxycarbonyl, ethyloxycarbonyl radicals,
- or R represents a radical

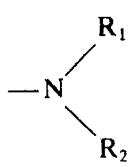

  in which $R_1$ and $R_2$, identical or different, represent a hydrogen atom, an alkyl radical containing 1—6 carbon atoms, or $R_1$ and $R_2$ form, with the nitrogen atom to which they are linked, a heterocycle chosen from the group formed by 1-piperidinyl, 1-piperazinyl, 4-methyl piperazin-1-yl, 1-pyridinium, N-morpholinyl, 1-pyrrolyl, 1-imidazolyl, 1-pyridazinium, 1-pyrimidinium,
- or R represents a quaternary ammonium group,
- or R represents a halogen atom,
- or R represents a 1,2-epoxyethyl or 2,2-dimethyl-1,2-epoxyethyl group or a radical resulting from the opening of this group by a nucleophilic reagent,
- or R represents a group

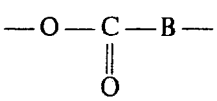

in which B represents either an alkyl or alkyloxy radical with at most 6 carbon atoms, or an aryl, aralkyl, aryloxy or aralkyloxy radical,

- or R represents a formyl radical, free or protected, a carboxyl radical, free, esterified or salified, a thiocyanate radical, a nitrile radical, an acyl radical or a carbamoyl radical, Ra represents a hydrogen atom or an acyl radical; the wavy line signifies that the products may be found in syn or anti form or in the form of a mixture of syn and anti;

as well as the salts of addition of these products with mineral or organic acids.

2. The products according to Claim 1, characterized in that R represents an alkyloxy, alkenyloxy, alkynyloxy, alkylthio, alkenylthio, alkynylthio, aryloxy, aralkyloxy, arylthio or aralkylthio group, possibly substituted by one or more of the following groups:

- alkyloxy or alkylthio with 1–6 carbon atoms,
- halogen,

or R represents the group

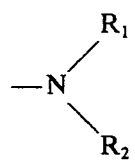

in which $R_1$ and $R_2$ have the significance indicated in Claim 1.

3. The products according to Claim 1, in which R represents:

either an alkyloxy radical possibly substituted by an alkyloxy radical or by a dialkylamino radical;
or an aryloxy or aralkyloxy radical possibly substituted by a halogen atom;
or an alkylthio or arylthio radical possibly substituted by a halogen atom;
or a dialkylamino radical;
or a halogen atom.

4. The products according to Claim 1, with the formula (I'):

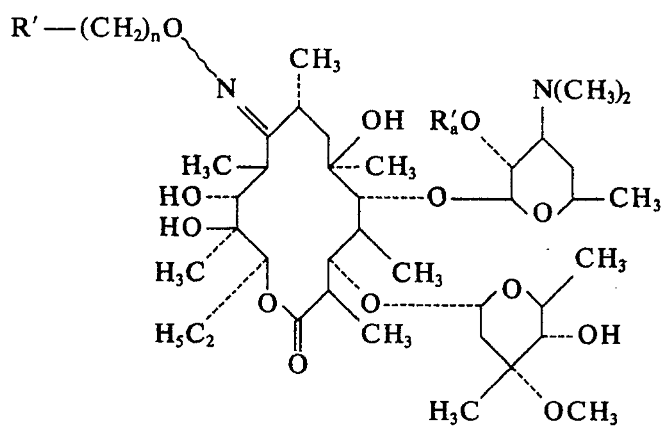

(I')

in which n represents an integer 1–6 and R' represents

- either an alkyloxy or alkyloxyalkyloxy radical with not more than 6 carbon atoms,
- or a phenyloxy or benzyloxy radical possibly substituted by a chlorine atom,
- or a dialkylamino radical;

R'a represents a hydrogen atom or an acyl radical with 2–6 carbon atoms, as well as their salts of addition with mineral or organic acids; the products with the formula (I') corresponding to the products with the formula (I) in which A represents a saturated alkylene radical $-(CH_2)_n-$, R has the values of R'and Ra has the values of R'a.

5. The products according to Claim 4, characterized in that R' represents:

- either a radical $CH_3-(CH_2)_{n1}-O-$ in which n1 represents a numeral 0–3,
- or a radical $CH_3-(CH_2)_{n1}-O-(CH_2)_{n2}-O-$ in which n1 has the preceding significance and n2 represents an integer 1–3.

6. The products according to Claim 4, characterized in that R′ represents a radical

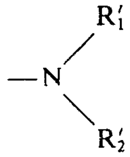

in which $R'_1$ and $R'_2$ represent an alkyl radical with 1—3 carbon atoms.

7. Any one of the products with the formula (I), as defined in Claim 1, the names of which follow:

- 9-[O-[(2-methoxyethoxy)-methyl]-oxime] of erythromycin,
- 9-[O-[2-(dimethylamino)-ethyl]-oxime] of erythromycin,
- 9-[O-[2-(diethylamino)-ethyl]-oxime] of erythromycin,

as well as the salts of addition of these products with mineral or organic acids.

8. Preparation process for the products with the formula (I) as defined in Claim 1, characterized in that either erythromycin with the formula (II):

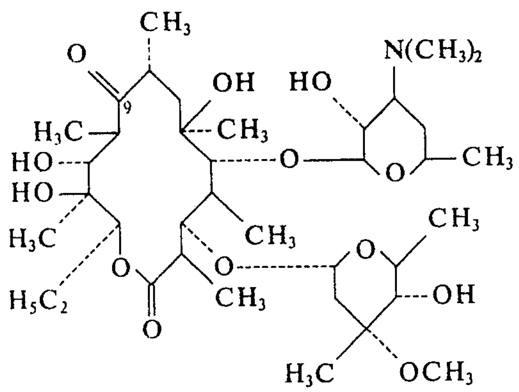

(II)

is treated, possibly in the presence of a base, with a product with the formula (III):

$$H_2N{-}O{-}A{-}R \qquad \text{(III)}$$

in which A and R are defined as in Claim 1, or with a strong acid salt of this product, so as to obtain a product with the formula ($I_1$) corresponding to a product with the formula (I) in which Ra represents a hydrogen atom,
or 9-oxime of erythromycin with the formula (IV):

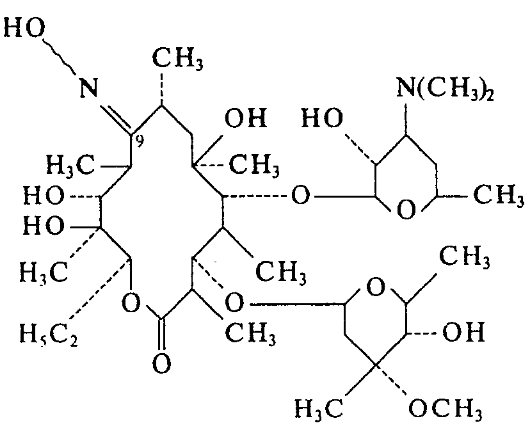

(IV)

in either of its isomeric forms, or in the form of a mixture, is treated, possibly in the presence of a base, with a product with the formula (V):

$$Hal{-}A{-}R \qquad \text{(V)}$$

in which A and R are defined as in Claim 1, and Hal represents a halogen atom, so as to obtain a product with the formula ($I_1$) corresponding to a product with the formula (I) in which Ra represents a hydrogen atom, which product with the formula ($I_1$), if applicable,

— when R represents a halogen atom or an alkoxy, alkenyloxy, alkynyloxy, alkylthio, alkenylthio or alkynylthio radical substituted by a halogen atom, may also be treated with a reagent with the formula

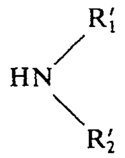

in which each of $R'_1$ and $R'_2$, identical or different, represents a hydrogen atom or an alkyl radical, linear or branched, containing 1—6 carbon atoms, or, with the nitrogen atom, form a heterocycle chosen from the group formed by 1-piperidinyl, 1-piperazinyl, 4-methylpiperazin-1-yl, 1-pyridinium, N-morpholinyl, 1-pyrrolyl, 1-imidazolyl, 1-pyridazinium, 1-pyrimidinium, so as to obtain a corresponding product with the formula ($I_2$), corresponding to a product with the formula (I) in which Ra represents a hydrogen atom and R represents a radical

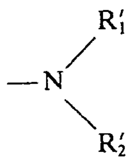

or an alkoxy, alkenyloxy, alkynyloxy, alkylthio, alkenylthio or alkynylthio radical substituted by a radical

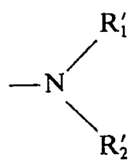

the radical

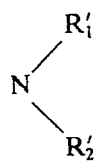

being as previously defined,

— when R represents a 1,2-epoxyethyl radical, possibly substituted, may also be treated with a nucleophilic reagent as previously defined, so as to obtain a corresponding product with the formula ($I_3$), corresponding to a product with the formula (I) in which Ra represents a hydrogen atom and R represents a radical resulting from the opening of the epoxy, which products with the formula (I), in which Ra represents a hydrogen atom, if applicable, are esterified so as to obtain products with the formula (I) in which Ra represents an acyl radical, and which products with the formula (I), if desired, are salified.

9. Preparation process according to Claim 8, characterized in that, either a product with the formula (II) is treated with a hydrochloride or a hydrobromide of a product with the formula (III) and in that the operation is carried out in the presence of an organic amino base or of an alkaline earth metal carbonate, or a product with the formula (IV) is treated with a product with the formula (V) in the presence of a base chosen from the group formed by carbonate and acid carbonate of sodium or of potassium, calcium carbonate, barium carbonate and sodium hydride.

10. As medicaments, the pharmaceutically acceptable products, as defined in Claim 1.

11. As medicaments, the pharmaceutically acceptable products as defined in any one of Claims 2 to 6.

12. As medicaments, any one of the pharmaceutically acceptable products with the formula (I) as defined in Claim 7.

13. The pharmaceutical compositions containing, as active principle, at least one of the medicaments according to any one of Claims 10 to 12.

**Claims for the contracting State: AT**

1. Process for preparing the derivatives of erythromycin with the formula:

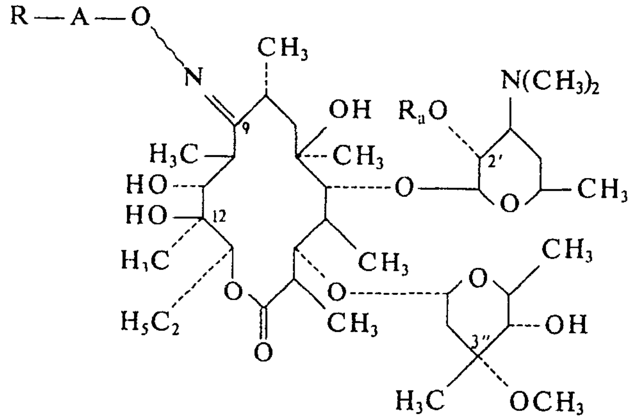

(I)

in which A represents an alkylene radical, linear or branched, with 1—6 carbon atoms and R represents

- either an alkyloxy, alkenyloxy or alkynyloxy radical with not more than 6 carbon atoms,
- or an alkylthio, alkenylthio or alkynylthio radical, with not more than 6 carbon atoms, the sulphur atom possibly being oxidized to sulphoxide or to sulphone,
- or an aryloxy, aralkyloxy radical,
- or an arylthio, aralkylthio radical, the sulphur atom possibly being oxidized to sulphoxide or to sulphone, each of these radicals is possibly substituted by one or more of the following groups: hydroxy, alkoxy or alkylthio, with 1—6 carbon atoms, vinyloxy, allyloxy, ethynyloxy, propargyloxy, vinylthio, allylthio, ethynylthio, propargylthio, halogen, amino, methylamino, dimethylamino, diethylamino, 1-piperazinyl, 4-methyl-piperazin-1-yl, 1-piperidinyl, 1-pyridinium, N-morpholino, 1-pyrrolyl, pyrrolidinyl, 1-imidazolyl, 1-pyridazinium, 1-pyrimidinium; the radicals: aryloxy, arylthio, aralkyloxy and aralkylthio, being moreover possibly substituted by methyl, ethyl, propyl, carbamoyl, aminomethyl, dimethylaminomethyl, aminoethyl, dimethylaminoethyl, carboxyl, methyloxycarbonyl, ethyloxycarbonyl radicals,
- or R represents a radical

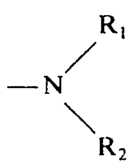

in which $R_1$ and $R_2$, identical or different, represent a hydrogen atom, an alkyl radical with 1—6 carbon atoms, or $R_1$ and $R_2$ form, with the nitrogen atom to which they are linked, a heterocycle chosen from the group formed by 1-piperidinyl, 1-piperazinyl, 4-methyl piperazin-1-yl, 1-pyridinium, N-morpholinyl, 1-pyrrolyl, 1-imidazolyl, 1-pyridazinium, 1-pyrimidinium,
- or R represents a quaternary ammonium group,
- or R represents a halogen atom,
- or R represents a 1,2-epoxyethyl or 2,2-dimethyl-1,2-epoxyethyl group or a radical resulting from the opening of this group by a nucleophilic reagent,
- or R represents a group

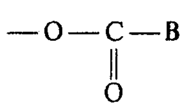

in which B represents either an alkyl or alkyloxy radical with not more than 6 carbon atoms, possibly substituted, or an aryl, aralkyl, aryloxy or aralkyloxy radical,
- or R represents a formyl radical, free or protected, a carboxyl radical, free, esterified or salified, a thiocyanate radical, a nitrile radical, an acyl radical or a carbamoyl radical, Ra represents a hydrogen atom or an acyl radical; the wavy line signifies that the products may be found in syn or anti form or in the form of a mixture of syn and anti;

as well as the salts of addition of these products with mineral or organic acids, characterized in that: either the erythromycin with the formula (II)

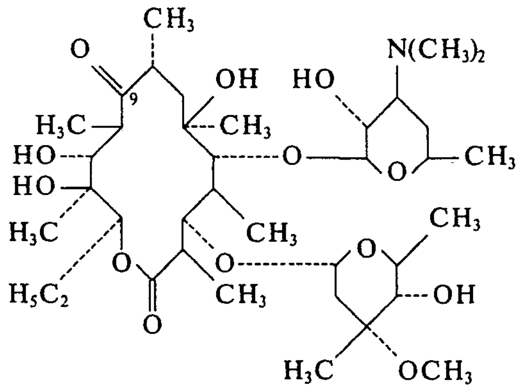

(II)

is treated, possibly in the presence of a base, with a product with the formula (III):

$$H_2N{-}O{-}A{-}R \qquad (III)$$

in which A and R are defined as previously, or with a strong acid salt of this product, so as to obtain a product with the formula ($I_1$), corresponding to a product with the formula (I) in which Ra represents a hydrogen atom,

or 9-oxime of erythromycin with the formula (IV):

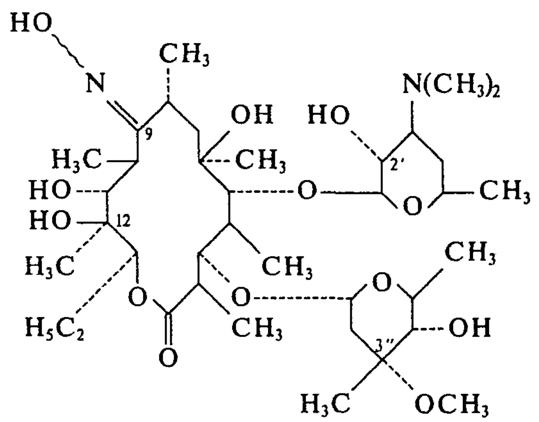

(IV)

in either of its isomeric forms, or in the form of a mixture, is treated, possibly in the presence of a base, with a product with the formula (V):

$$Hal{-}A{-}R \qquad (V)$$

in which A and R are defined as previously and Hal represents a halogen atom, so as to obtain a product with the formula ($I_1$), corresponding to a product with the formula (I) in which Ra represents a hydrogen atom, which product with the formula ($I_1$), if applicable,

- when R represents a halogen atom or an alkoxy, alkenyloxy, alkynyloxy, alkylthio, alkenylthio or alkynylthio radical substituted by a halogen atom, may also be treated with a reagent with the formula

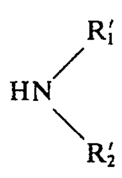

in which $R'_1$ and $R'_2$, identical or different, represent a hydrogen atom or an alkyl radical, linear or branched, containing 1—6 carbon atoms, or form, with the nitrogen atom, a heterocycle possibly containing another heteroatom and possibly substituted, so as to obtain a corresponding product with the formula ($I_2$), corresponding to a product with the formula (I) in which Ra represents a hydrogen atom and R

represents a radical

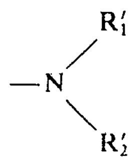

or an alkoxy, alkenyloxy, alkynyloxy, alkylthio, alkenylthio or alkynylthio radical, substituted by a radical

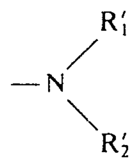 the radical 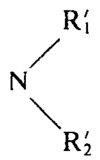

being as previously defined,

- when R represents a 1,2-epoxyethyl radical possibly substituted, may also be treated with a nucleophilic reagent as previously defined, so as to obtain a corresponding product with the formula ($I_3$), corresponding to a product with the formula (I) in which Ra represents a hydrogen atom and R represents a radical resulting from the opening of the epoxy, which products with the formula (I) in which Ra represents a hydrogen atom, if applicable, are esterified so as to obtain products with the formula (I) in which Ra represents an acyl radical, and which products with the formula (I), if desired, are salified.

2. Preparation process according to Claim 1, characterized in that, either a product with the formula (II) is treated with a hydrochloride or a hydrobromide of a product with the formula (III) and in that the operation is carried out in the presence of an organic amino base or of an alkaline earth metal carbonate, or a product with the formula (IV) is treated with a product with the formula (V) in the presence of a base chosen from the group formed by carbonate and acid carbonate of sodium or of potassium, calcium carbonate, barium carbonate and sodium hydride.

3. Process according to Claim 1 or 2, characterized in that a product with the formula (III) or (V) is used at the start in which R represents an alkyloxy, alkenyloxy, alkynyloxy, alkylthio, alkenylthio, alkynylthio, aryloxy, aralkyloxy, arylthio or aralkylthio group possibly substituted by one or more of the following groups:

- alkyloxy or alkylthio with 1–6 carbon atoms,
- halogen,

or R represents the group

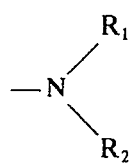

in which $R_1$ and $R_2$ have the significance indicated in Claim 2.

4. Process according to claim 1 or 2, characterized in that a product with the formula (III) or (V) is used at the start in which R represents:

either an alkyloxy radical possibly substituted by an alkyloxy radical or by a dialkylamino radical;
or an aryloxy or aralkyloxy radical possibly substituted by a halogen atom;
or an alkylthio or arylthio radical possibly substituted by a halogen atom;
or a dialkylamino radical;
or a halogen atom.

5. Process according to Claim 1 or 2, characterized in that a product with the formula (III) or (V) is used at the start in which R represents:

- either an alkyloxy or alkyloxyalkyloxy radical with not more than 6 carbon atoms,
- or a phenyloxy or benzyloxy radical possibly substituted by a chlorine atom,
- or a dialkylamino radical and A represents a saturated alkylene radical $-(CH_2)_n-$.

6. Process according to claim 1 or 2, characterized in that a product with the formula (III) or (V) is used at the start in which R represents:

- either a radical $CH_3-(CH_2)_{n1}-O-$ in which n1 represents a numeral 0 to 3,
- or a radical $CH_3-(CH_2)_{n1}-O-(CH_2)_{n2}-O-$ in which n1 has the preceding significance and n2 represents an integer 1 to 3.

7. Process according to Claim 1 or 2, characterized in that a product with the formula (III) or (V) is used at the start in which R represents a radical

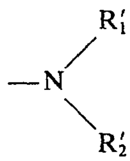

in which $R'_1$ and $R'_2$ represent an alkyl radical containing 1—3 carbon atoms.

8. Process according to Claim 1 or 2, characterized in that a product with the formula (III) or (V) is used at the start, chosen in such a way that any one of the products with the formula (I) is prepared, as defined in Claim 1, the names of which follow:

- 9-[O-[(2-methoxyethoxy)-methyl]-oxime] of erythromycin,
- 9-[O-[2-(dimethylamino)-ethyl]-oxime] of erythromycin,
- 9-[O-[2-(diethylamino)-ethyl]-oxime] of erythromycin,

as well as the salts of addition of these products with mineral or organic acids.